# EUROPEAN PATENT SPECIFICATION

(11) **EP 4 105 213 B1**
(45) Date of publication and mention of the grant of the patent: **15.04.2026**
(21) Application number: 20914046.6
(22) Date of filing: 14.07.2020
(51) Int. Cl.: C07D 471/04, A61K 31/519, A61K 31/5377, A61P 35/00

(54) **PYRIDO[3,4-D]PYRIMIDINE DERIVATIVE AND THERAPEUTIC PHARMACEUTIC COMPOSITION COMPRISING SAME**
PYRIDO[3,4-D]PYRIMIDINDERIVAT UND DIESES ENTHALTENDE THERAPEUTISCHE PHARMAZEUTISCHE ZUSAMMENSETZUNG
DÉRIVÉ DE PYRIDO[3,4-D]PYRIMIDINE ET COMPOSITION PHARMACEUTIQUE THÉRAPEUTIQUE LE COMPRENANT

(30) Priority: 15.01.2020 KR 20200005292
(43) Date of publication of application: 21.12.2022
(73) Proprietor: Pharos Ibio Co., Ltd., Gyeonggi-do 14059 (KR)
(72) Inventor: SIM, Tae Bo, Seoul 02792 (KR); PARK, Chan Sun, Seoul 02792 (KR); CHOI, Seung Hye, Seoul 02792 (KR); CHO, Han Na, Seoul 02792 (KR); SENGUPTA, Sandip, Seoul 02792 (KR); SHIN, In Jae, Seoul 02792 (KR); HUR, Woo Young, Seoul 02792 (KR); KUMAR, Jayprakash Narayan, Seoul 02792 (KR)
(74) Representative: Weickmann & Weickmann PartmbB
(86) International application number: PCT/KR2020/009259
(87) International publication number: WO 2021/145521

(56) References cited:
- WO-A1-2014/037750
- WO-A1-2019/223766
- WO-A1-2019/242587
- WO-A1-2020/172093
- CN-A- 109 745 325
- US-A1- 2019 276 434

## Description

### Technical Field

The present invention provides a compound selected from among a novel pyrido[3,4-d]pyrimidine derivative having protein kinase inhibitory activity, a pharmaceutically acceptable salt thereof, a hydrate thereof, and a stereoisomer thereof, and a method for producing the compound, and a pharmaceutical composition for preventing, alleviating or treating cancer containing the compound.

### Background Art

Protein kinases are enzymes catalyzing phosphorylation reactions that transfer a gamma-phosphate group from ATP to the hydroxyl groups of tyrosine, serine and threonine residues of proteins. These protein kinases are involved in cell metabolism, gene expression, cell growth, cell differentiation and cell division, and an important role in cell signaling.

Protein kinases account for about 2% of the eukaryotic genome, and there are about 518 protein kinases in the human genome. Protein kinases are classified into tyrosine protein kinases that phosphorylate tyrosine, and serine/threonine kinases that phosphorylate serine and threonine. More than about 90 protein kinases are tyrosine kinases, and are divided into receptor tyrosine kinases (RTKs) and non-receptor tyrosine kinases (NRTKs). Receptor tyrosine kinases are membrane proteins which have domains capable of receiving growth factors on the cell surface and have active sites capable of phosphorylating tyrosine residues in the cytoplasm. Non-receptor tyrosine kinases are single tyrosine kinase domains present in the nucleus and cytoplasm, and phosphorylate tyrosine residues by receiving signals even though they are not receptors.

Protein kinases are molecular switches and the transition between active and inactive states thereof in cells should be smoothly regulated. When they are abnormally regulated, they excessively activate intracellular signaling, resulting in uncontrollable cell division and proliferation. In addition, abnormal activation of protein kinases by gene mutation, amplification and overexpression is associated with the development and progression of various tumors, and thus plays a decisive role in the growth and metastasis of cancer cells. Typical examples of protein kinases that are abnormally regulated include EGFR, VEGFR, PDGFRB, c-KIT, ABL1, SRC, BRAF, FGFR, BTK, SYK, ALK, MET, CDK, MEK, mTOR, JAK, LCK, PLK, RSK, LYN, FMS, TIE2, RET, AKT, MAP, FAK, DDR, FLT3, and FES. In particular, since receptor tyrosine kinases are mainly involved in external signaling pathways for cell growth and signaling pathways for internal responses, inhibition thereof may also lead to cancer cell growth inhibition and death.

Due to these characteristics, inhibition of kinase activity has attracted attention as a major target for the development of anticancer drugs, and studies on the development of low-molecular-weight organic compounds targeting various kinases have been actively conducted.

Examples of kinase inhibitors include Gleevec^{®} (imatinib, Novartis) which is a Bcr-Abl and PDGFR tyrosine kinase inhibitor, Herceptin^{®} (trastuzumab, Genentech) which is an Her-2 antibody, Iressa^{®} (gefitinib, AstraZeneca) which is an EGFR inhibitor, Nexavar^{®} (sorafenib, Bayer) which is an inhibitor of Raf, VEGFR, KIT, RET, PDGFR-B and FLT-3, Zelboraf^{®} (vemurafenib, Roche) which is a BRAF inhibitor, Erbitux^{®} (cetuximab, Imclone) which is an EGFR antibody, Tarceva^{®} (erlotinib, Genentech/Roche) which is an EGFR inhibitor, and Sutent^{®} (sunitinib, Pfizer) which is a KDR inhibitor. They have been approved by the FDA for use as anticancer drugs for leukemia, breast cancer, non-small cell lung cancer, liver cancer, malignant melanoma, colorectal cancer and the like, and are widely used as first-line standard therapy due to their excellent therapeutic efficacy. In addition, various compounds are in clinical trials.

Meanwhile, acute myeloid leukemia (AML) is one of fatal blood diseases in which blood cells differentiate abnormally and proliferate continuously. More than 16% of acute myeloid leukemia (AML) patients have a point-mutated RAS (small G protein) protein, and NRAS mutations account for the majority (10% or more) of RAS kinases. For this reason, NRAS G protein has been considered as a promising drug target for the treatment of AML. When the protooncogene RAS is mutated, RAS is continuously activated (gain-of-function), and various signaling pathways downstream of RAS are activated to accelerate cancer cell growth.

Over the last 40 years, RAS point mutations or key signaling molecules downstream of RAS have been proposed as targets. However, they did not lead to *in vivo* experiments and clinical trials due to the complexity and compensatory effect of mutant RAS signaling pathways. For example, selumetinib (AZD 6244), which inhibits MEK, a key molecule downstream of RAS, showed no therapeutic effect in all three AML patients with a NRAS mutant gene in phase 2 clinical trials. In addition, in an attempt to find targets, RNA interference screening was used to identify proteins (TBK1, STK33 and GATA2) which are genetically in a synthetic lethal relationship with KRAS mutation. However, this also failed to achieve clinical therapeutic effects. In particular, in the case of STK33, it was proven through cell-based pharmacological screening at the preclinical stage that therapeutic strategies using KRAS mutation and synthetic lethal principles cannot be established. In addition, in recent years, cell-based pharmacological screening was used to identify a compound (GNF 7) that selectively inhibits the RAS mutant signaling pathway, and the inhibitory effects thereof in a preclinical leukemia model were confirmed. The mechanism of action of the GNF 7 compound is to simultaneously inhibit two kinases, GCK and ACK1, which specifically contribute to cell growth downstream of the RAS mutant signaling pathway. This compound also actually exhibited its efficacy in cell samples from AML patients with NRAS mutants. Inhibitors against two kinases, GCK and ACK1, are known to be effective for the treatment of cancer diseases caused by NRAS mutation, such as melanoma, colorectal cancer, thyroid cancer, and various blood cancers.

Meanwhile, International Patent Publication No. WO 2005-011597 (Patent Document 1) discloses an amide compound having 3,4-dihydropyrimido[4,5-d]pyrimidin-2(1H)-one as a parent nucleus structure, and discloses that the compound is effective for the treatment of diseases or conditions caused by the activities of kinases such as Abl or BCR-Abl. However, Patent Document 1 merely discloses that the amide compound is useful as a therapeutic agent for chronic myelogenous leukemia (CML) based on the BCR-Abl inhibitory activity thereof.

### [Prior Art Documents]

### [Patent Documents]

(Patent Document 1) International Patent Publication No. WO 2015-011597

### [Non-Patent Documents]

(Non-Patent Document 1) Choi HG, Ren P, Adrian F, et al. A type-II kinase inhibitor capable of inhibiting the T315I "gatekeeper" mutant of Bcr-Abl. J. Med. Chem., 2010; 53(15): 5439-5448.
(Non-Patent Document 2) Nonami, A.; Sattler, M.; Weisberg, E.; Liu, Q.; Zhang, J.; Patricelli, M. P.; Christie, A. L.; Saur, A. M.; Kohl, N. E.; Kung, A. L.; Yoon, H.; Sim, T.; Gray, N. S.; Griffin, J. D., Identification of novel therapeutic targets in acute leukemias with NRAS mutations using a pharmacologic approach. Blood 2015, 125 (20), 3133-43.
(Non-Patent Document 3) Luo T, Masson K, Jaffe JD, et al. STK33 kinase inhibitor BRD-8899 has no effect on KRAS-dependent cancer cell viability. Proc Natl Acad Sci USA. 2012;109(8): 2860-2865.
(Non-Patent Document 4) Jain N, Curran E, Iyengar NM, et al. Phase II study of the oral MEK inhibitor selumetinib in advanced acute myelogenous leukemia: a University of Chicago phase II consortium trial. Clin Cancer Res. 2014; 20(2): 490-498.
(Non-Patent Document 5) Johnson, D. B.; Smalley, K. S.; Sosman, J. A., Molecular pathways: targeting NRAS in melanoma and acute myelogenous leukemia. Clin Cancer Res 2014,20(16), 4186-92.
(Non-Patent Document 6) H Cho, I Shin, E Ju, et al. First SAR Study for Overriding NRAS Mutant Driven Acute Myeloid Leukemia. J. Med. Chem. 2018, 61 (18), 8353-8373

### DISCLOSURE

### Technical Problem

Therefore, an object of the present invention is to provide a novel pyrido[3,4-d]pyrimidine derivative compound having protein kinase inhibitory activity.

Another object of the present invention is to provide a pharmaceutical composition useful for the treatment, prevention and alleviation of cancer disease, the pharmaceutical composition containing, as an active ingredient, a novel pyrido[3,4-d]pyrimidine derivative compound, a pharmaceutically acceptable salt thereof, a hydrate thereof, a solvate thereof, or a stereoisomer thereof.

Still another object of the present invention is to provide a therapeutic agent for a cancer disease caused by NRAS mutation, the therapeutic agent containing, as an active ingredient, a novel pyrido[3,4-d]pyrimidine derivative compound, a pharmaceutically acceptable salt thereof, a hydrate thereof, a solvate thereof, or a stereoisomer thereof.

### Technical Solution

### IN ORDER TO ACHIEVE THE ABOVE OBJECTS, THE PRESENT INVENTION PROVIDES A COMPOUND SELECTED FROM AMONG A PYRIDO[3,4-D]PYRIMIDINE DERIVATIVE COMPOUND DEFINED IN CLAIM 1, A PHARMACEUTICALLY ACCEPTABLE SALT THEREOF, A HYDRATE THEREOF, AND A STEREOISOMER THEREOF.

### Advantageous Effects

The compound according to the present invention has excellent ability to inhibit the activity of protein kinase such as ABL1, ABL2/ARG, ACK1, ARAF, BLK, BMX/ETK, BRAF, BRK, BTK, c-Kit, c-Src, CSK, DDR1, DDR2, EPHA1, EPHA2, EPHA3, EPHA4, EPHA5, EPHA6,EPHA7, EPHA8, EPHB1, EPHB2, EPHB3, EPHB4, ERBB2/HER2, ERBB4/HER4, FGFR1, FGFR2, FGFR3, FGR, FLT1/VEGFR1, FLT4/VEGFR3, FMS, FRK/PTK5, FYN,GCK/MAP4K2, HCK, HGK/MAP4K4, JAK1, JAK2, KDR/VEGFR2, KHS/MAP4K5, LCK, LYN, LYN B, MEKK1, MLK4, MYLK3, NEK5, P38a/MAPK14, P38b/MAPK11, PDGFRa, PDGFRb, PEAK1, RET, RIPK2, RIPK4, SIK2, SRPK1, TAOK3/JIK, TLK1, TNK1, TXK, TYK2, YES/YES1, YSK4/MAP3K19, or ZAK/MLTK. Thus, the compound may be used for the purpose of treating, preventing and alleviating cancer disease caused by abnormal cell growth.

The compound according to the present invention, a pharmaceutically acceptable salt thereof, or a hydrate thereof, and a pharmaceutical composition for preventing or treating cancer containing the same as an active ingredient exhibit high inhibitory activity and an antiproliferative effect selectively against cancer cells while showing low cytotoxicity, and thus may be effectively used for the prevention or treatment of cancer. The references to methods of treatment in the subsequent paragraphs of this description are to be interpreted as references to the compounds, pharmaceutical compositions and medicaments of the present invention for use in a method for treatment of the human (or animal) body by therapy.

Examples of cancer diseases that may be treated, prevented and alleviated by treatment with the compound according to the present invention include stomach cancer, lung cancer, liver cancer, colorectal cancer, small intestine cancer, pancreatic cancer, brain cancer, bone cancer, melanoma, breast cancer, sclerosing adenosis, uterine cancer, cervical cancer, head and neck cancer, esophageal cancer, thyroid cancer, parathyroid cancer, kidney cancer, sarcoma, prostate cancer, urethral cancer, bladder cancer, blood cancer (including leukemia, multiple myeloma, and myelodysplastic syndrome), lymphoma (Hodgkin's disease, and non-Hodgkin's lymphoma), psoriasis, or fibroadenoma.

In particular, the compound according to the present invention has excellent inhibitory activity against two kinases, GCK and ACK1, and thus is effective for the treatment of cancer disease caused by NRAS mutation, such as melanoma, colorectal cancer, thyroid cancer, or acute myeloid leukemia (AML).

### Best Mode

Since all numbers, values and/or expressions referring to quantities of components, reaction conditions, and mixtures used in the present specification are subject to various uncertainties of measurement encountered in obtaining such values, unless otherwise indicated, all are to be understood as modified in all instances by the term "about." Where a numerical range is disclosed herein, such a range is continuous, inclusive of both the minimum and maximum values of the range as well as every value between such minimum and maximum values, unless otherwise indicated. Still further, where such a range refers to integers, every integer between the minimum and maximum values of such a range is included, unless otherwise indicated.

In the present specification, where a range is stated for a parameter, it will be understood that the parameter includes all values within the stated range, inclusive of the stated endpoints of the range. For example, a range of 5 to 10 will be understood to include the values 5, 6, 7, 8, 9, and 10, as well as any sub-range such as 6 to 10, 7 to 10, 6 to 9, and 7 to 9, and also include any value and range between the integers which are reasonable in the context of the range stated, such as 5.5, 6.5, 7.5, 5.5 to 8.5 and 6.5 to 9. For example, a range of "10% to 30%" will be understood to include the values 10%, 11%, 12%, 13%, etc., and all integers up to and including 30%, as well as any sub-range such as 10% to 15%, 12% to 18%, 20% to 30%, etc., and also include any value between the integers which are reasonable in the context of the range stated, such as 10.5%, 15.5%, 25.5%, etc.

Hereinafter, the present invention will be described in detail.

The present inventors have conducted extensive studies to solve the above-described problems, and as a result, have developed an anticancer compound exhibiting excellent inhibitory activity against cancer cells, particularly a pyrido[3,4-d]pyrimidine derivative compound useful for the prevention or treatment of cancer as a selective kinase activity inhibitor, a pharmaceutically acceptable salt thereof, a hydrate thereof, and a stereoisomer thereof, and a pharmaceutical composition for preventing or treating cancer containing the same as an active ingredient. A method for producing the same is described but not claimed.

One aspect of the present invention provides a compound selected from among a pyrido[3,4-d]pyrimidine derivative compound as defined in claim 1, a pharmaceutically acceptable salt thereof, a hydrate thereof, and a stereoisomer thereof.

The common structure of the compounds of the present invention is shown in [Formula 2] 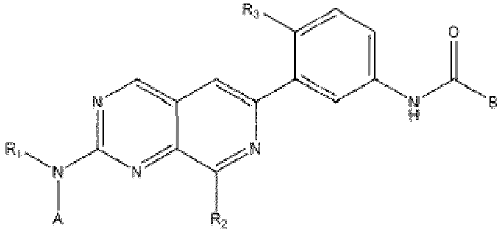 wherein
B is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C(O)-(C₁-C₁₃ alkyl) ;
A is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or C(O)- (C₁-C₁₃ alkyl), or A together with a nitrogen atom to which R₁ is attached forms a 4- to 7-membered saturated, unsaturated or aromatic ring, which may optionally contain at least one of N, O, NH, C=N, C=O and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₁ is hydrogen, a C₁-C₁₃ alkyl group, a C₃-C₁₀ cyclyl group, or a C₃-C₁₀ heterocyclyl group, or R₁ together with a nitrogen atom to which A is attached forms a 4- to 7-membered saturated, unsaturated or aromatic ring, which may optionally contain at least one of N, O, NH, C=N, C=O and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₂ and R₃ are each hydrogen, a hydroxyl group, a halogen group, a C₁-C₆ alkyl group, a C₁-C₆ alkenyl group, a C₆-C₁₀ aryl group, a C₃-C₁₀ heteroaryl group, or a C₃-C₁₀ heterocyclyl group;
R₄ is hydrogen or a C₁-C₆ alkyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amide group (- (C=O) NR₅R₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group unsubstituted or substituted with a halogen or C₃-C₁₀ heterocyclyl group, a C₁-C₃ alkoxy group unsubstituted or substituted with a halogen or C₃-C₁₀ heterocyclyl group, C₆-C₁₀ phenoxy, an amino group (-NR₅R₆), an amide group (-(C=O)NR₅R₆), a C₆-C₁₀ aryl group, a C₃-C₁₀ heteroaryl group, and a C₃-C₁₀ heterocyclyl group;
R₅ and R₆ contain at least one selected from the group consisting of hydrogen, a C₁-C₆ alkyl group, a C₁-C₆ alkenyl group, a C₁-C₆ alkynyl group, a C₆-C₁₀ aryl group, a C₃-C₁₀ heteroaryl group, and a C₃-C₁₀ heterocyclyl group; and
the C₃-C₁₀ heteroaryl group and the C₃-C₁₀ heterocyclyl group contain at least one heteroatom selected from the group consisting of N, O and S.

The general formula (2) is provided for illustration purposes while the compound of the invention is selected from the group consisting of the following Compound Nos. 1 to 26:
(Compound No. 1): *N*-(3-(8-chloro-2-((6-methylpyridin-3-yl)amino)pyrido[3,4-d]pyrimidin-6-yl)-4-methylphenyl)-3-(trifluoromethyl)benzamide;
(Compound No. 2): *N*-(4-methyl-3-(2-((6-methylpyridin-3-yl)amino)-8-vinylpyrido[3,4-*d*]pyrimidin-6-yl)phenyl)-3-(trifluoromethyl)benzamide;
(Compound No. 3): *N*-(3-(8-ethyl-2-((6-methylpyridin-3-yl)amino)pyrido[3,4-d]pyrimidin-6-yl)-4-methylphenyl)-3-(trifluoromethyl)benzamide;
(Compound No. 4): *N*-(4-methyl-3-(2-((6-methylpyridin-3-yl)amino)pyrido[3,4-d]pyrimidin-6-yl)phenyl)-3-(trifluoromethyl)benzamide;
(Compound No. 5): *N*-(3-(2-((2-hydroxyethyl)amino)-8-methoxypyrido[3,4-*d*]pyrimidin-6-yl)-4-methylphenyl)-3-(trifluoromethyl)benzamide;
(Compound No. 6): *N*-(3-(2-(cyclopropylamino)-8-methoxypyrido[3,4-*d*]pyrimidin-6-yl)-4-methylphenyl)-3-(trifluoromethyl)benzamide;
(Compound No. 7): *N*-(3-(8-methoxy-2-morpholinopyrido[3,4-d]pyrimidin-6-yl)-4-methylphenyl)-3-(trifluoromethyl)benzamide;
(Compound No. 8): *N*-(3-(8-methoxy-2-((6-methylpyridin-3-yl)amino)pyrido[3,4-d]pyrimidin-6-yl)-4-methylphenyl)-3-(trifluoromethyl)benzamide;
(Compound No. 9): *N*-(3-(8-methoxy-2-((6-morpholinopyridin-3-yl)amino)pyrido[3,4-d]pyrimidin-6-yl)-4-methylphenyl)-3-(trifluoromethyl)benzamide;
(Compound No. 10): *N*-(3-(8-methoxy-2-((2-methoxy-4-morpholinophenyl)amino)pyrido[3,4-*d*]pyrimidin-6-yl)-4-methylphenyl)-3-(trifluoromethyl)benzamide;
(Compound No. 11): *N*-(3-(2-((4-(4-ethylpiperazin-1-yl)phenyl)amino)-8-methoxypyrido[3,4-*d*]pyrimidin-6-yl)-4-methylphenyl)-3-(trifluoromethyl)benzamide;
(Compound No. 12): *N*-(4-methyl-3-(2-((6-methylpyridin-3-yl)amino)pyrido[3,4-*d*]pyrimidin-6-yl)phenyl)-4-(morpholinemethyl)-3-(trifluoromethyl)benzamide;
(Compound No. 13): 3-(4-methyl-1*H*-imidazol-1-yl) -N-(4-methyl-3-(2-((6-methylpyridin-3-yl)amino)pyrido[3,4-*d*]pyrimidin-6-yl)phenyl)-5-(trifluoromethyl)benzamide;
(Compound No. 14): *N*-(4-methyl-3-(2-((6-methylpyridin-3-yl)amino)pyrido[3,4-*d*]pyrimidin-6-yl)phenyl)-3-morpholino-5-(trifluoromethyl)benzamide;
(Compound No. 15): 4-(2,4-dimethyl-1*H*-imidazo-1-yl)-*N*-(4-methyl-3-(2-((6-methylpyridin-3-yl)amino)pyrido[3,4-*d*]pyrimidin-6-yl)phenyl)-3-(trifluoromethyl)benzamide;
(Compound No. 16): 4-(4-hydroxypiperidin-1-yl)-*N*-(4-methyl-3-(2-((6-methylpyridin-3-yl)amino)pyrido[3,4-*d*]pyrimidin-6-yl)phenyl)-3-(trifluoromethyl)benzamide;
(Compound No. 17): *N*-(3-(2-aminopyrido[3,4-*d*]pyrimidin-6-yl)-4-methylphenyl)-3-(trifluoromethyl)benzamide;
(Compound No. 18): *N*-(3-(2-((2-hydroxyethyl)amino)pyrido[3,4-*d*]pyrimidin-6-yl)-4-methylphenyl)-3-(trifluoromethyl)benzamide;
(Compound No. 19): *N*-(3-(2-(cyclicpropylamino)-pyrido[3,4-*d*]pyrimidin-6-yl)-4-methylphenyl)-3-(trifluoromethyl)benzamide;
(Compound No. 20): *N*-(4-methyl-3-(2-(phenylamino)pyrido[3,4-*d*]pyrimidin-6-yl)phenyl)-3-(trifluoromethyl)benzamide;
(Compound No. 21): *N*-(4-methyl-3-(2-((1-methyl-1*H-*pyrazo-4-yl)amino)pyrido[3,4-*d*]pyrimidin-6-yl)phenyl)-3-(trifluoromethyl)benzamide;
(Compound No. 22): *N*-(3-(2-((1,3-dimethyl-1*H*-pyrazol-5-yl)amino)pyrido[3,4-*d*]pyrimidin-6-yl)-4-methylphenyl)-3-(trifluoromethyl)benzamide;
(Compound No. 23): *N*-(4-methyl-3-(2-((4-methylpyridin-3-yl)amino)pyrido[3,4-*d*]pyrimidin-6-yl)phenyl)-3-(trifluoromethyl)benzamide;
(Compound No. 24): *N*-(3-(2-((2,6-dimethylpyridin-3-yl)amino)pyrido[3,4-*d*]pyrimidin-6-yl)-4-methylphenyl)-3-(trifluoromethyl)benzamide;
(Compound No. 25): *N-*(3-(2-((4-(4-ethylpiperazin-1-yl)phenyl)amino)pyrido[3,4-*d*]pyrimidin-6-yl)-4-methylphenyl)-3-(trifluoromethyl)benzamide; and
(Compound No. 26): *N*-(3-(2-((3-(4-ethylpiperazin-1-yl)phenyl)amino)pyrido[3,4-*d*]pyrimidin-6-yl)-4-methylphenyl)-3-(trifluoromethyl)benzamide.

In the definition of substituents in the present invention, the term "'alkyl" refers to an aliphatic hydrocarbon radical. The alkyl may be saturated alkyl that does not contain an alkenyl or alkynyl moiety, or unsaturated alkyl that contains at least one alkenyl or alkynyl moiety. The term "alkenyl" refers to a group containing at least one carbon-carbon double bond, and the term "alkynyl" refers to a group containing at least one carbon-carbon triple bond. The alkyl may be cyclic, branched or straight-chain when used alone or in combination.

The term "aryl" as used herein, either alone or in combination with another radical, refers to a carbocyclic aromatic monocyclic group containing 6 carbon atoms, which may be further fused to a second 5- or 6-membered carbocyclic group which may be aromatic, saturated or unsaturated. Examples of aryl include, but are not limited to, phenyl, indanyl, 1-naphthyl, 2-naphthyl, and tetrahydronaphthyl. Aryl may be connected to another group at a suitable position on the aromatic ring.

The term "alkoxy" refers to an alkyl group attached via an oxygen atom to another group (i.e., -O-alkyl). The alkoxy group may be unsubstituted or substituted with one or more suitable substituents. Examples of the alkoxy group include, but are not limited to, (C₁-C₆) alkoxy groups, for example, -O-methyl, -O-ethyl, -O-propyl, -O-isopropyl, -O-2-methyl-1-propyl, -O-2-methyl-2-propyl, -O-2-methyl-1-butyl, -O-3-methyl-1-butyl, -I-2-methyl-3-butyl, -O-2,2-dimethyl-1-propyl, -O-2-methyl-1-pentyl, 3-O-methyl-1-pentyl, -O-4-methyl-1-pentyl, -O-2-methyl-2-pentyl, -O-3-methyl-2-pentyl, -O-4-methyl-2-pentyl, -O-2,2-dimethyl-1-butyl, -O-3,3-dimethyl-butyl, -O-2-ethyl-1-butyl, -O-butyl, -O-isobutyl, -O-t-butyl, -O-pentyl, -O-isopentyl, -O-neopentyl, and -O-hexyl.

The term "phenoxy" refers to a phenyl group attached via an oxygen atom to another group (i.e., -O-aryl). The phenoxy group may be unsubstituted or substituted with one or more halogens, alkyl groups, aryl groups or heteroaryl groups, but is not limited thereto.

The term "amino group" refers to an alkyl group attached via a nitrogen atom to another group (i.e., -NH- or -N-alkyl). The amino group may be unsubstituted or substituted with one or more suitable substituents. Examples of the amino group include, but are not limited to, (C₁-C₆) amino groups, for example, -NH-methyl, -NH-ethyl, -NH-propyl, -NH-isopropyl, -NH-2-methyl-1-propyl, -NH-2-methyl-2-propyl, -NH-2-methyl-1-butyl, -NH-3-methyl-1-butyl, -NH-2-methyl-3-butyl, -NH-2,2-dimethyl-1-propyl, -NH-2-methyl-1-pentyl, 3-NH-methyl-1-pentyl, -NH-4-methyl-1-pentyl, -NH-2-methyl-2-pentyl, -NH-3-methyl-2-pentyl, -NH-4-methyl-2-pentyl, -NH-2,2-dimethyl-1-butyl, -NH-3,3-dimethyl-butyl, - NH-2-ethyl-1-butyl, -NH-butyl, -NH-isobutyl, -NH-t-butyl, - NH-pentyl, -NH-isopentyl, -NH-neopentyl, -NH-hexyl, -N,N-dimethyl, -N-methyl-N-ethyl, -N-methyl-N-propyl, -N-methyl-isopropyl, -N-methyl-N-butyl, -N-methyl-N-isobutyl, -N-methyl-N-pentyl, -N-methyl-N-isopentyl, N-methyl-N-hexyl, N-methyl-N-isohexyl, -N,N-diethyl, -N-ethyl-N-propyl, -N-ethyl-N-isopropyl, -N-ethyl-N-butyl, -N-ethyl-N-isobutyl, - N-ethyl-N-pentyl, -N-ethyl-N-isopentyl, -N-ethyl-N-hexyl, - N-ethyl-N-isohexyl, -N,N-dipropyl, -N-propyl-N-isopropyl, - N-propyl-N-butyl, -N-propyl-N-isobutyl, -N-propyl-N-pentyl, -N-propyl-N-isopentyl, -N-propyl-N-hexyl, -N-propyl-N-isohexyl, -N,N-dibutyl, -N-butyl-N-isobutyl, -N-butyl-N-pentyl, -N-butyl-N-isopentyl, -N-butyl-N-hexyl, -N-butyl-N-isohexyl, -N,N-dipentyl, -N-pentyl-N-hexyl, -N-pentyl-N-isohexyl, and -N,N-dihexyl.

The term "halogen group" refers to fluorine, chlorine, bromine or iodine.

The term "heterocyclyl group" refers to a heteroaromatic compound containing at least one heteroatom selected from the group consisting of N, O and S, unless otherwise stated. Preferably, examples of the heterocyclyl group may include, but are not limited to, a pyrrolidine group, a furan group, a morpholine group, a piperazine group and a piperidine group, more preferably a pyrrolidine group, a piperidine group, a piperazine group, and a morpholine group.

The term "heteroaryl group" refers to a heteroaromatic compound containing at least one heteroatom selected from the group consisting of N, O and S, unless otherwise stated. Preferably, examples of the heteroaryl group may include, but are not limited to, a pyridine group, a pyrazine group, a pyrimidine group, a pyridazine group, a pyrazole group, an imidazole group, a triazole group, an indole group, an oxadiazole group, a thiadiazole group, a quinolone group, an isoquinoline group, an isoxazole group, an oxazole group, a thiazolyl group, and a pyrrole group.

The compounds according to the present invention are as follows:
(Compound No. 1): *N*-(3-(8-chloro-2-((6-methylpyridin-3-yl)amino)pyrido[3,4-*d*]pyrimidin-6-yl)-4-methylphenyl)-3-(trifluoromethyl)benzamide 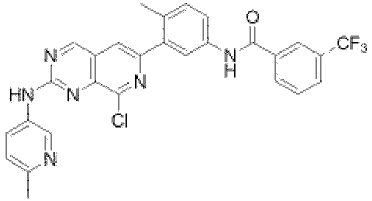
(Compound No. 2): *N*-(4-methyl-3-(2-((6-methylpyridin-3-yl)amino)-8-vinylpyrido[3,4-*d*]pyrimidin-6-yl)phenyl)-3-(trifluoromethyl)benzamide 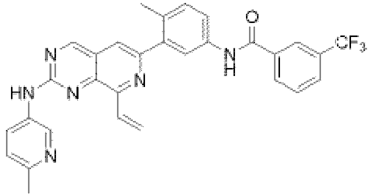
(Compound No. 3): *N*-(3-(8-ethyl-2-((6-methylpyridin-3-yl)amino)pyrido[3,4-*d*]pyrimidin-6-yl)-4-methylphenyl)-3-(trifluoromethyl)benzamide 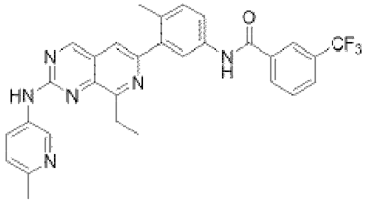
(Compound No. 4): *N*-(4-methyl-3-(2-((6-methylpyridin-3-yl)amino)pyrido[3,4-*d*]pyrimidin-6-yl)phenyl)-3-(trifluoromethyl)benzamide 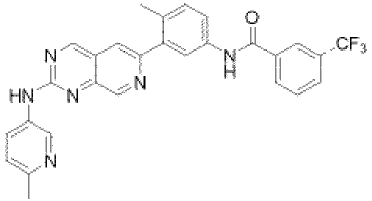
(Compound No. 5): *N*-(3-(2-((2-hydroxyethyl)amino)-8-methoxypyrido[3,4-*d*]pyrimidin-6-yl)-4-methylphenyl)-3-(trifluoromethyl)benzamide 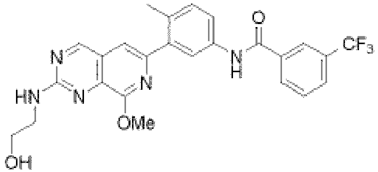
(Compound No. 6): *N*-(3-(2-(cyclopropylamino)-8-methoxypyrido[3,4-*d*]pyrimidin-6-yl)-4-methylphenyl)-3-(trifluoromethyl)benzamide 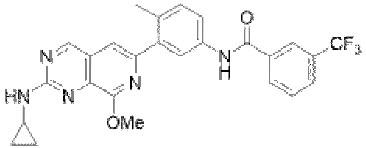
(Compound No. 7): *N*-(3-(8-methoxy-2-morpholinopyrido[3,4-d]pyrimidin-6-yl)-4-methylphenyl)-3-(trifluoromethyl)benzamide 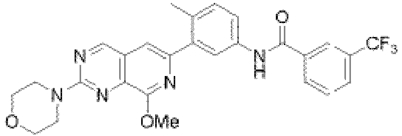
(Compound No. 8): *N*-(3-(8-methoxy-2-((6-methylpyridin-3-yl)amino)pyrido[3,4-d]pyrimidin-6-yl)-4-methylphenyl)-3-(trifluoromethyl)benzamide 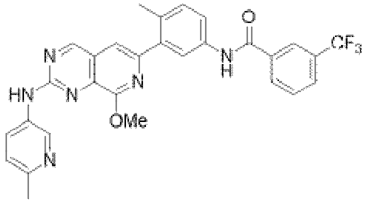
(Compound No. 9): *N*-(3-(8-methoxy-2-((6-morpholinopyridin-3-yl)amino)pyrido[3,4-d]pyrimidin-6-yl)-4-methylphenyl)-3-(trifluoromethyl)benzamide 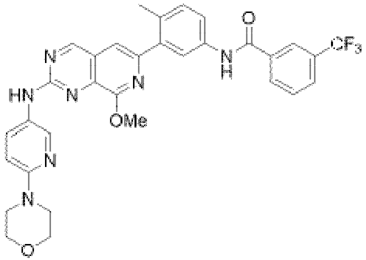
(Compound No. 10): *N*-(3-(8-methoxy-2-((2-methoxy-4-morpholinophenyl)amino)pyrido[3,4-d]pyrimidin-6-yl)-4-methylphenyl)-3-(trifluoromethyl)benzamide 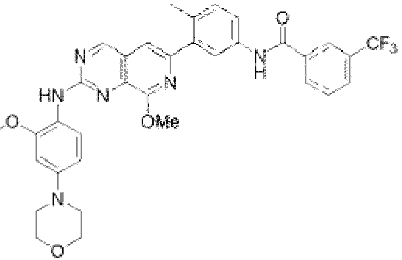
(Compound No. 11): *N*-(3-(2-((4-(4-ethylpiperazin-1-yl)phenyl)amino)-8-methoxypyrido[3,4-*d*]pyrimidin-6-yl)-4-methylphenyl)-3-(trifluoromethyl)benzamide 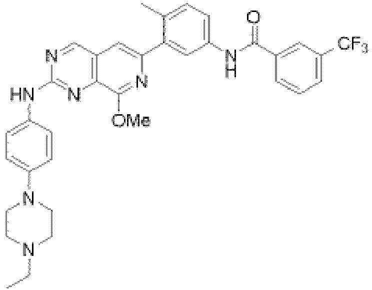
(Compound No. 12): *N*-(4-methyl-3-(2-((6-methylpyridin-3-yl)amino)pyrido[3,4-*d*]pyrimidin-6-yl)phenyl)-4-(morpholinemethyl)-3-(trifluoromethyl)benzamide 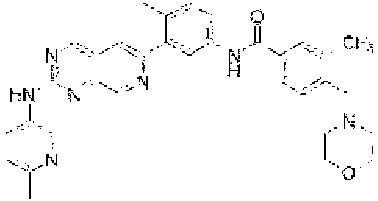
(Compound No. 13): 3-(4-methyl-1*H*-imidazol-1-yl)-N-(4-methyl-3-(2-((6-methylpyridin-3-yl)amino)pyrido[3,4-*d*]pyrimidin-6-yl)phenyl)-5-(trifluoromethyl)benzamide 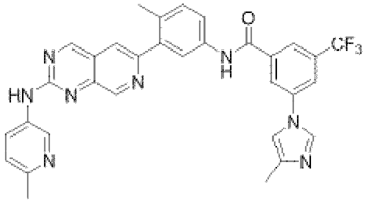
(Compound No. 14): *N*-(4-methyl-3-(2-((6-methylpyridin-3-yl)amino)pyrido[3,4-*d*]pyrimidin-6-yl)phenyl)-3-morpholino-5-(trifluoromethyl)benzamide 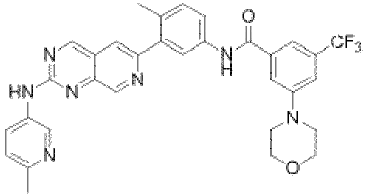
(Compound No. 15): 4-(2,4-dimethyl-1*H*-imidazo-1-yl)-*N*-(4-methyl-3-(2-((6-methylpyridin-3-yl)amino)pyrido[3,4-*d*]pyrimidin-6-yl)phenyl)-3-(trifluoromethyl)benzamide 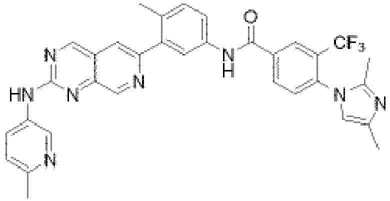
(Compound No. 16): 4-(4-hydroxypiperidin-1-yl)-*N*-(4-methyl-3-(2-((6-methylpyridin-3-yl)amino)pyrido[3,4-*d*]pyrimidin-6-yl)phenyl)-3-(trifluoromethyl)benzamide 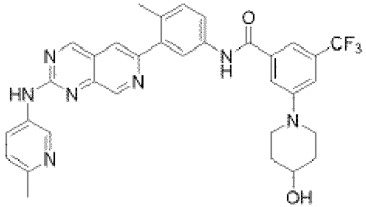
(Compound No. 17): *N*-(3-(2-aminopyrido[3,4-d]pyrimidin-6-yl)-4-methylphenyl)-3-(trifluoromethyl)benzamide 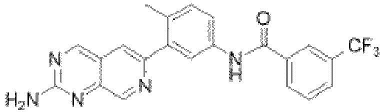
(Compound No. 18) : *N*-(3-(2-((2-hydroxyethyl)amino)pyrido[3,4-d]pyrimidin-6-yl)-4-methylphenyl)-3-(trifluoromethyl)benzamide 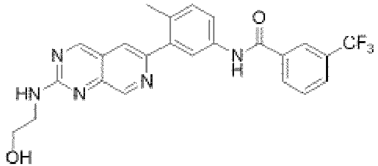
(Compound No. 19): *N*-(3-(2(cyclicpropylamino)-pyrido[3,4-*d*]pyrimidin-6-yl)-4-methylphenyl)-3-(trifluoromethyl)benzamide 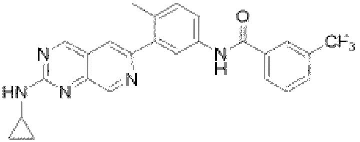
(Compound No. 20): *N*-(4-methyl-3-(2-(phenylamino)pyrido[3,4-d]pyrimidin-6-yl)phenyl)-3-(trifluoromethyl)benzamide 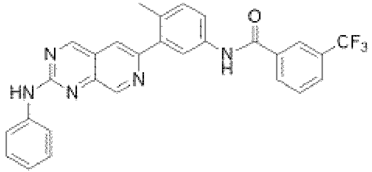
(Compound No. 21): *N*-(4-methyl-3-(2-((1-methyl-1*H-*pyrazo-4-yl)amino)pyrido[3,4-*d*]pyrimidin-6-yl)phenyl)-3-(trifluoromethyl)benzamide 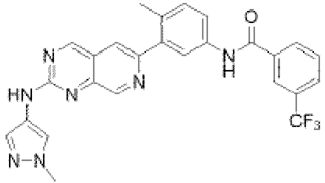
(Compound No. 22): *N*-(3-(2-((1,3-dimethyl-1*H*-pyrazol-5-yl)amino)pyrido[3,4-*d*]pyrimidin-6-yl)-4-methylphenyl)-3-(trifluoromethyl)benzamide 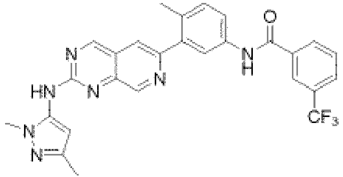
(Compound No. 23): *N*-(4-methyl-3-(2-((4-methylpyridin-3-yl)amino)pyrido[3,4-d]pyrimidin-6-yl)phenyl)-3-(trifluoromethyl)benzamide 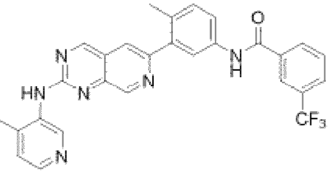
(Compound No. 24): *N*-(3-(2-((2,6-dimethylpyridin-3-yl)amino)pyrido[3,4-*d*]pyrimidin-6-yl)-4-methylphenyl)-3-(trifluoromethyl)benzamide 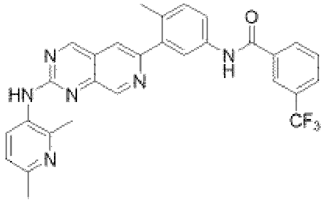
(Compound No. 25): *N*-(3-(2-((4-(4-ethylpiperazin-1-yl)phenyl)amino)pyrido[3,4-*d*]pyrimidin-6-yl)-4-methylphenyl)-3-(trifluoromethyl)benzamide 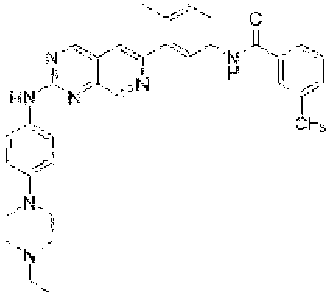
(Compound No. 26): *N*-(3-(2-((3-(4-ethylpiperazin-1-yl)phenyl)amino)pyrido[3,4-*d*]pyrimidin-6-yl)-4-methylphenyl)-3-(trifluoromethyl)benzamide

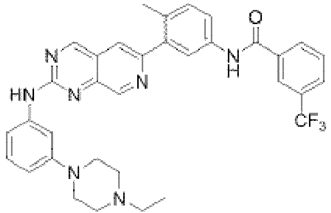

The compound of the present invention may be used in the form of a pharmaceutically acceptable salt derived from an inorganic acid or an organic acid. A preferred salt may be formed with one or more selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, and toluenesulfonic acid.

The compound according to the present invention or a pharmaceutically acceptable salt thereof may include a hydrate and a solvate. The hydrate may refer to one formed by the combination of the compound of the invention with a water molecule.

Another aspect of the present invention provides a pharmaceutical composition for preventing, alleviating or treating cancer containing, as an active ingredient, a compound according to the present invention, a pharmaceutically acceptable salt thereof, a hydrate thereof, and a stereoisomer thereof.

The pharmaceutical composition according to the present invention has excellent ability to inhibit the activity of protein kinase. Specific examples of the protein kinase include ABL1, ABL2/ARG, ACK1, ARAF, BLK, BMX/ETK,BRAF, BRK, BTK, c-Kit, c-Src, CSK, DDR1, DDR2, EPHA1, EPHA2, EPHA3, EPHA4, EPHA5, EPHA6, EPHA7, EPHA8, EPHB1, EPHB2, EPHB3, EPHB4, ERBB2/HER2,ERBB4/HER4, FGFR1, FGFR2, FGFR3, FGR, FLT1/VEGFR1, FLT4/VEGFR3, FMS, FRK/PTK5, FYN, GCK/MAP4K2, HCK, HGK/MAP4K4, JAK1, JAK2, KDR/VEGFR2,KHS/MAP4K5, LCK, LYN, LYN B, MEKK1, MLK4, MYLK3, NEK5, P38a/MAPK14, P38b/MAPK11, PDGFRa, PDGFRb, PEAK1, RET, RIPK2, RIPK4, SIK2, SRPK1, TAOK3/JIK,TLK1, TNK1, TXK, TYK2, YES/YES1, YSK4/MAP3K19, and ZAK/MLTK.

Thus, the pharmaceutical composition of the present invention may be used for the purpose of treating, preventing and alleviating cancer disease caused by abnormal cell growth. Examples of cancer diseases that may be prevented, treated and alleviated by treatment with the pharmaceutical composition according to the present invention include stomach cancer, lung cancer, liver cancer, colorectal cancer, small intestine cancer, pancreatic cancer, brain cancer, bone cancer, melanoma, breast cancer, sclerosing adenosis, uterine cancer, cervical cancer, head and neck cancer, esophageal cancer, thyroid cancer, parathyroid cancer, kidney cancer, sarcoma, prostate cancer, urethral cancer, bladder cancer, blood cancer (including leukemia, multiple myeloma, and myelodysplastic syndrome), lymphoma (Hodgkin's disease, and non-Hodgkin's lymphoma), psoriasis, or fibroadenoma.

In particular, the pharmaceutical composition of the present invention has inhibitory activity against two kinases, GCK and ACK1, and thus is effective for the treatment of cancer diseases caused by NRAS mutation, for example, melanoma, colorectal cancer, thyroid cancer, or various blood cancers. In addition, the compound of the invention exhibits inhibitory activity against the proliferation of the NRAS mutant cell line (OCI-AML3) without showing inhibitory activity against the Ba/F3 (parental) cell line, and thus is particularly effective as a therapeutic agent for treating acute myeloid leukemia (AML).

Preferably, the cancer may be cancer caused by a protein kinase. More preferably, the protein kinase may be at least one selected from among GCK and ACK1.

Another aspect of the present invention provides a pharmaceutical composition for preventing, alleviating or treating cancer, the pharmaceutical composition containing, as an active ingredient, any one compound selected from among the above-described compounds of the invention.

In another aspect of the present invention, there is provided a pharmaceutical composition for preventing, alleviating or treating cancer, wherein the cancer is caused by NRAS mutation.

In another aspect of the present invention, there is provided a pharmaceutical composition for preventing, alleviating or treating cancer, wherein the pharmaceutical composition is applied to patients with NRAS mutation.

In another aspect of the present invention, there is provided a pharmaceutical composition for preventing, alleviating or treating cancer, wherein the cancer is at least one selected from the group consisting of melanoma, colorectal cancer, thyroid cancer, and blood cancer.

In another aspect of the present invention, there is provided a pharmaceutical composition for preventing, alleviating or treating cancer, wherein the cancer is acute myeloid leukemia (AML).

In another aspect of the present invention, there is provided a pharmaceutical composition for preventing, alleviating or treating cancer, wherein the pharmaceutical composition is administered to a patient with NRAS G12D.

The pharmaceutical composition may be applied to experimental animals such as mice, rabbits, rats, guinea pigs, or hamsters, or primates including humans, but is not limited thereto. Preferably, the pharmaceutical composition may be applied to primates including humans, more preferably humans.

In the present invention, the term "treating" or "treatment" may be used in a sense including alleviation or amelioration of symptoms, reduction of the range of disease, delay or alleviation of disease progression, amelioration, alleviation or stabilization of disease conditions, partial or complete recovery, prolonging of survival, and other beneficial therapeutic outcomes. It is understood that any references to methods of treatment herein are to be interpreted as references to the compounds, pharmaceutical compositions and medicaments of the present invention for use in such methods of treatment.

In addition, the treatment of cancer as used in the present specification refers to treatment of all cancer cell types, and the term "cancer" also includes angiogenesis of endothelial cells and mitosis thereof (solid tumors, tumor metastases and benign tumors). Examples of the cancer include, but are not limited to, breast cancer, ovarian cancer, cervical cancer, prostate cancer, testicular cancer, genitourinary tract cancer, esophageal cancer, laryngeal cancer, glioblastoma, stomach cancer, skin cancer, keratoacanthoma, lung cancer, squamous cell carcinoma, large cell carcinoma, small cell carcinoma, lung adenocarcinoma, bone cancer, colon cancer, adenoma, pancreatic cancer, adenocarcinoma, thyroid cancer, follicular adenocarcinoma, undifferentiated cancer, papillary cancer, seminoma, melanoma, sarcoma, bladder cancer, liver cancer, bile duct cancer, kidney cancer, myeloid disease, lymphoid disease, Hodgkin's disease, hair cell cancer, oral cancer, pharyngeal (laryngeal) cancer, lip cancer, tongue cancer, small intestine cancer, colorectal cancer, large intestine cancer, rectal cancer, brain cancer, central nervous system cancer, leukemia, angioma, trachoma, and pyogenic granuloma.

Depending on the aspect and method of use of the pharmaceutical composition of the present invention, the content of the compound of the invention, a pharmaceutically acceptable salt thereof, or a hydrate thereof, which is an active ingredient, may be appropriately selected and adjusted for use by those skilled in the art.

For example, the pharmaceutical composition may contain the compound of the invention, a pharmaceutically acceptable salt thereof, or a hydrate thereof in an amount of 0.1 to 10 wt%, more preferably 0.5 to 5 wt% by weight, based on the total weight of the composition.

The compound represented of the invention, a pharmaceutically acceptable salt thereof, or a hydrate thereof may be contained alone in the pharmaceutical composition or may also be contained together with a pharmacologically acceptable carrier, excipient, diluent or inactive ingredient.

Examples of the pharmaceutically acceptable carrier, excipient or diluent include, but are not limited to, at least one selected from the group consisting of lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, dextrin, calcium carbonate, propylene glycol, liquid paraffin, and physiological saline. In addition, any conventional carrier, excipient or diluent may also be used. In addition, the pharmaceutical composition may further contain a conventional filler, extender, binder, disintegrant, anti-aggregating agent, lubricant, wetting agent, pH-adjusting agent, nutrient, vitamin, electrolyte, alginic acid and its salt, pectic acid and its salt, protective colloid, glycerin, fragrance, emulsifier or preservative.

The compound according to the present invention or a pharmaceutically acceptable salt thereof may be co-administered with other anticancer drugs for treating cancer or tumors, thus enhancing the therapeutic effects of the anticancer drugs.

Specifically, the pharmaceutical composition may further contain one or more other anticancer drugs or other therapeutic agents known to be effective for the treatment or prevention of cancer, in addition to the active ingredient, and may be used in combination therapy in which they are applied simultaneously or at different times. For example, other anticancer drugs or other therapeutic agents that may be applied to the combination therapy may include, but are not limited to, one or more compounds selected from the group consisting of Gleevec^{®} (imatinib), Sutent^{®} (sunitinib), Herceptin^{®} (Trastuzumab), Velcade^{®} (Bortezomib) dexamethasone, Nexavar^{®} (Sorafenib), aromatase inhibitors, and kinase inhibitors.

The mode of administration of the pharmaceutical composition may be oral or parenteral. For example, the pharmaceutical composition may be administered through various routes, including oral, transdermal, subcutaneous, intravenous or intramuscular routes. In addition, the formulation of the composition may vary depending on the method of use thereof, and the composition may be formulated using a method well known in the art to which the present invention pertains, so as to provide rapid, sustained or delayed release of the active ingredient after administration to a mammal. In general, solid preparations for oral administration include tablets, troches, soft or hard capsules, pills, powders, and granules, and these formulations may be prepared by mixing one or more excipients, for example, starch, calcium carbonate, sucrose, lactose, or gelatin. In addition to simple excipients, lubricants such as magnesium stearate and talc may also be used. Liquid formulations for oral administration include suspensions, liquids for internal use, emulsions, and syrups, which may contain various excipients, for example, wetting agents, sweetening agents, fragrances, preservatives, and the like, in addition to water and liquid paraffin, which are commonly used simple diluents. Dosage forms for parenteral administration include cream, lotions, ointments, plasters, liquids, solutions, aerosols, fluid extracts, elixirs, infusions, sachets, patches, or injections. When the dosage form for parenteral administration is a dosage form for injection, it may preferably be in the form of an isotonic aqueous solution or suspension.

The pharmaceutical composition may further contain a sterilizing agent, a preservative, a stabilizer, a wetting agent or an emulsifying agent, adjuvants such as a salt and/or buffer for regulating osmotic pressure, and other therapeutically useful substances, and may be formulated according to a conventional mixing, granulation or coating method. In addition, the pharmaceutical composition may be formulated using an appropriate method known in the art.

In addition, the dosage of the pharmaceutical composition may be determined in consideration of the mode of administration, the patient's age and sex, the patient's disease severity and condition, the in vivo absorption rate of the active ingredient, the rate of inactivation, and drugs to be used in combination, and may be administered once or several times. The active ingredient of the pharmaceutical composition may preferably administered to mammals including humans once or several times a day by an oral or parenteral route at a dose of 0.001 to 100 mg/kg body weight/day, preferably 0.01 to 35 mg/kg body weight/day.

Another aspect described herein is a method for treating cancer, the method comprising a step of administering a therapeutically effective amount of the compound of the invention, a pharmaceutically acceptable salt thereof, or a hydrate thereof.

Preferably, the treatment method may further comprise a step of identifying a patient in need of the prevention or treatment of the cancer, before the administering step.

In the present invention, the term "therapeutically effective amount" refers to an amount of the active ingredient for a mammal, which is effective for the prevention or treatment of cancer. The therapeutically effective amount may be adjusted depending on various factors, including the kind of disease, the severity of the disease, the kinds and contents of the active ingredient and other ingredients contained in the composition, the type of formulation, the patient's age, weight, general health status, sex and diet, the time of administration, the route of administration, the blood clearance of the composition, the duration of treatment, and drugs that are used concurrently. Preferably, as described above, the active ingredient may be administered once or several times a day by an oral or parenteral route at a dose of 0.001 to 100 mg/kg body weight/day, preferably 0.01 to 35 mg/kg body weight/day.

In addition, the present document describes a method for producing the pyrido[3,4-d]pyrimidine derivative compound represented by Formula 2, a pharmaceutically acceptable salt thereof, or a hydrate thereof.

### Synthesis Examples: Production of pyrido[3,4-d]pyrimidine derivative compounds of Formula 2

### Synthesis Example 1. Production of N-(3-(8-chloro-2-((6-methylpyridin-3-yl)amino)pyrido[3,4-d]pyrimidin-6-yl)-4-methylphenyl)-3-(trifluoromethyl)benzamide (Compound No. 1)

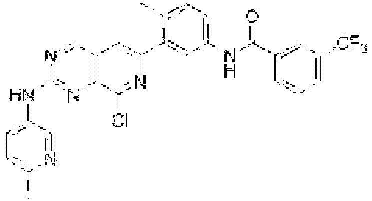

### Step 1: Methyl 5-bromo-2-(methylthio)pyrimididine-4-carboxylate

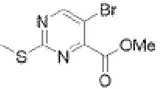

Acetyl chloride (1.1 eq., 10.5 mL) was dissolved in methanol (200 ml) at 0°C. After stirring at 0°C for 10 minutes, 5-bromo-2-(methylthio)pyrimidine-4-carboxylic acid (25 g, 1 eq.) was slowly added over 15 minutes. The reaction solution was refluxed for 4 hours and cooled. After completion of the reaction, the reaction solution was concentrated under reduced pressure. The concentrate was diluted with dichloromethane, and then washed with a saturated aqueous solution of sodium bicarbonate and then washed with brine. The organic layer was dried with magnesium sulfate and concentrated. The concentrate was recrystallized from ethyl acetate and hexane and then filtered to obtain the title compound (20 g, 76% yield) which was yellow in color. ¹H-NMR: (400 MHz, CDCl₃): δ (ppm): 8.68 (1H, s), 3.98 (3H, s), 2.54 (3H, s). LCMS (ESI): 263 (M + H)⁺.

### step 2: Trimethyl((2-methyl-5-nitrophenyl)ethynyl)silane

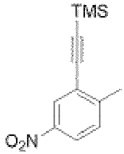

In a round bottom flask, 2-bromo-1-methyl-4-nitrobenzene (15 g, 1 eq.), diisopropylethylamine (24 mL, 2 eq.), Pd(PPh₃)₄ (4 g, 0.05 eq.), cuprous iodide (1.3 g, 0.1 eq.) and trimethylsilylacetylene (12 mL, 1.2 eq.) were dissolved in dimethylformamide (50 mL). After stirring at 80°C for 4 hours, the reaction solution was filtered through celite. The filtrate was diluted with ethyl acetate and then washed with brine. The organic layer was dried with magnesium sulfate and concentrated. The concentrate was purified by chromatography (2% ethyl acetate/hexane) to obtain the title compound (10.5 g, 65% yield) which was brown in color. ¹H-NMR: (400 MHz, CD₆CO): δ (ppm): 8.18 (1H, s), 8.14 (1H, d, J = 8.5 Hz), 7.58 (1H, d, *J* = 8.5 Hz), 2.54 (3H, s), 0.28 (9H, s).

### Step 3: 2-ethynyl-1-methyl-4-nitrobenzene

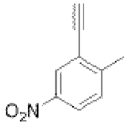

Calcium carbonate (15 g, 2.5 eq.) and methanol (100 mL) were placed in a round bottom flask, and trimethyl((2-methyl-5-nitrophenyl)ethynyl)silane (10.0 g, 1 eq.) was added slowly thereto dropwise. After completion of the reaction, the reaction solution was filtered through celite. The filtrate was diluted with ethyl acetate, and then washed with a saturated aqueous solution of ammonium chloride and then washed with brine. The organic layer was dried with magnesium sulfate and concentrated. The concentrate was purified by chromatography (2% ethyl acetate/hexane) to obtain the title compound (5.5 g, 80% yield) which was black in color. ¹H-NMR (400 MHz, CD₆CO): δ (ppm):8.23 (1H, s), 8.15 (1H, d, *J* = 8.5 Hz), 7.57 (1H, d, *J* = 8.5 Hz), 4.13 (1H, s), 2.55 (3H, s).

### step 4: Methyl 5-((2-methyl-5-nitrophenyl)ethynyl)-2-(methylthio)pyrimidine-4-carboxylate

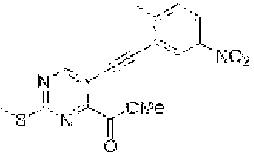

Methyl 5-bromo-2-(methylthio)pyrimididine-4-carboxylate (5 g, 1 eq.), 2-ethynyl-1-methyl-4-nitrobenzene (4.5 g, 1.2 eq.), PdCl₂(PPh₃)₂ (670 mg, 0.05 eq.) and cuprous iodide (362 mg, 0.1 eq.) were placed in a round bottom flask. Triethylamine as a solvent was added thereto, and then the reaction solution was stirred at 80°C for 25 hours. After completion of the reaction, the reaction solution was filtered through celite. The filtrate was diluted with dichloromethane and then washed with brine. The organic layer was dried with magnesium sulfate and concentrated. The concentrate was purified by chromatography (5% ethyl acetate/hexane) to obtain the title compound (5 g, 77% yield) which was white in color. ¹H-NMR: (400 MHz, CD₆CO): δ (ppm): 9.06 (1H, s), 8.35 (1H, s), 8.19 (1H, d, *J* = 8.5 Hz), 7.64 (1H, d, *J* = 8.5 Hz),4.02 (3H, s), 2.69 (3H, s), 2.63 (3H, s). LCMS (ESI): 344 (M + H)⁺.

### step 5: 5-((2-methyl-5-nitrophenyl)ethynyl)-2-(methylthio)pyrimidine-4-carboxylic acid

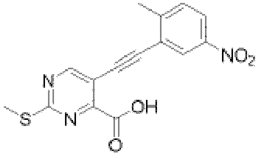

Methyl 5-((2-methyl-5-nitrophenyl)ethynyl)-2-(methylthio)pyrimidine-4-carboxylate (5 g, 1 eq.) was placed in a round bottom flask and dissolved slowly by the addition of tetrahydrofuran (50 mL). At 0°C, a saturated aqueous solution (40 mL) of 2 N sodium hydroxide was added slowly thereto dropwise. The reaction solution was stirred for 24 hours, and then tetrahydrofuran was removed under reduced pressure. The residue was adjusted to a pH of 3 to 4 using hydrogen chloride, and the formed solid was filtered and washed with water (2×50 mL). The washed solid was dried to obtain the title compound (3.4 g, 97% yield) which was yellow in color. ¹H-NMR: (400 MHz, DMSO-*d*₆) : δ (ppm) : 9.08 (1H, s), 8.27 (1H, s), 8.17 (1H, d, *J* = 8.5 Hz), 7.61 (1H, d, *J* = 8.5 Hz), 3.50 (1H, brs), 2.59 (3H, s), 2.57 (3H, s). LCMS (ESI): 330 (M + H)⁺.

### step 6: 6-(2-methyl-5-nitrophenyl)-2-(methylthio)-8H-pyrano[3,4-d]pyrimidin-8-one

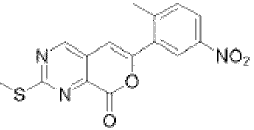

5-((2-methyl-5-nitrophenyl)ethynyl)-2-(methylthio)pyrimidine-4-carboxylic acid (3.4 g, 1 eq.) was placed in a round bottom flask and dissolved by the addition of toluene (25 mL). *p*-toluenesulfonic acid monohydrate (500 mg, 0.8 eq.) was added thereto, followed by stirring at 110°C for 7 hours. After completion of the reaction, the reaction solution was diluted with 10% methanol/dichloromethane, and then washed with a saturated aqueous solution of sodium bicarbonate and then washed with brine. The organic layer was dried with magnesium sulfate and concentrated. The concentrate was purified by chromatography (50% ethyl acetate/hexane) to obtain the title compound (2.4 g, 71% yield) which was yellow in color. ¹H-NMR: (400 MHz, CD₆CO): δ (ppm): 9.20 (1H, s), 8.45 (1H, s), 8.29 (1H, d, J = 8.5 Hz), 7.71 (1H, d, J = 8.5 Hz), 7.18 (1H, s), 2.70 (3H, s), 2.68 (3H, s). LCMS (ESI): 330 (M + H)⁺.

### step 7: 6-(2-methyl-5-nitrophenyl)-2-(methylthio)pyrido[3,4-d]pyrimidin-8(7H)-one

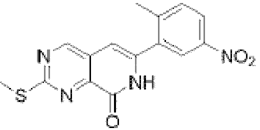

6-(2-methyl-5-nitrophenyl)-2- (methylthio) -8H-pyrano[3,4-d]pyrimidin-8-one (2.4 g, 1 eq.), acetic acid (25 mL) and ammonium acetate (5.6 g, 10 eq.) were placed in a round bottom flask and stirred at 90°C for 12 hours. After acetic acid was removed under reduced pressure, the residue was diluted with 20% isopropanol/chloroform. The dilution was washed with a saturated aqueous solution of sodium bicarbonate and then washed with brine. The organic layer was dried with magnesium sulfate and concentrated to obtain the title compound (2.2 g, 92% yield) which was brown in color. ¹H-NMR: (400 MHz, DMSO-*d*₆): δ (ppm): 12.34 (1H, brs), 9.22 (1H,s), 8.72 (1H, s), 8.23 (1H, d, *J* = 8.5 Hz), 7.63 (1H, d, *J* = 8.5 Hz), 6.67 (1H, s), 2.63 (3H, s), 2.44 (3H, s). LCMS (ESI): 329 (M + H)⁺. LCMS (ESI): 329 (M + H)⁺.

### step 8: 6-(2-methyl-5-nitrophenyl)-2-(methylsulfonyl)pyrido[3,4-d]pyrimidin-8(7H)-one

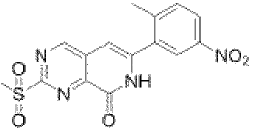

In a round bottom flask, 6-(2-methyl-5-nitrophenyl)-2- (methylthio) pyrido [3,4-*d*]pyrimidin-8 (7*H*) -one (2.2 g, 1 eq.) was dissolved in dichloromethane (25 mL). At 0°C, 3-chloroperbenzoic acid (3.5 g, 3 eq.) was added to the solution, followed by stirring at room temperature for 24 hours. After completion of the reaction, the reaction solution was diluted with 10% methanol/dichloromethane, and washed with a saturated aqueous solution of sodium bicarbonate and then washed with brine. The organic layer was dried with magnesium sulfate and concentrated. The concentrate was purified by chromatography (0-10% methanol/dichloromethane) to obtain the title compound (1.2 g, 50% yield) that was yellow in color. ¹H-NMR: (400 MHz, CD₆CO): δ (ppm): 11.30 (1H, brs), 9.58 (1H, s), 8.37 (1H, s), 8.30 (1H, d, *J* = 8.5 Hz), 7.71 (1H, d, J = 8.5Hz), 6.92 (1H, s), 3.44 (3H, s), 2.60 (3H, s). LCMS (ESI): 361 (M + H)⁺.

### Step 9: 6-(2-methyl-5-nitrophenyl)-2-((6-methylpyridin-3-yl)amino)pyrido[3,4-d]pyrimidin-8(7H)-one

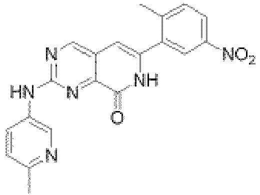

6-(2-methyl-5-nitrophenyl)-2-(methylsulfonyl)pyrido[3,4-*d*]pyrimidin-8(7*H*)-one (1.2 g, 1 eq.), *N*-(6-methylpyrimidin-3-yl)formamide (685 mg, 1.5 eq.), cesium carbonate (5.5 g, 5 eq.) and dimethyl sulfoxide (10 mL) were placed in a round bottom flask. The mixture was stirred at 90°C for 2 hours. After completion of the reaction, the reaction solution was filtered through celite. The filtrate was diluted with ethyl acetate and then washed with brine. The organic layer was dried with magnesium sulfate and concentrated. The concentrate was purified by chromatography (0-10% methanol/dichloromethane) to obtain the title compound (400 mg, 31% yield) that was yellow in color. ¹H-NMR: (400 MHz, DMSO-*d*₆) : δ (ppm) : 11.99 (1H, brs), 10.23 (1H, s), 9.14 (1H, s), 8.97 (1H, s), 8.30 (1H, d, J = 8.4 Hz), 8.23(1H, d, *J* = 8.5 Hz), 8.21 (1H, s), 7.63 (1H, d, *J* = 8.5 Hz), 7.22 (1H, d, *J* = 8.4 Hz), 6.62 (1H, s), 2.45 (3H, s), 2.43 (3H, s). LCMS (ESI): 389 (M + H)⁺.

### step 10: 8-chloro-6-(2-methyl-5-nitrophenyl)-N-(6-methylpyridin-3-yl)pyrido[3,4-d]pyrimidine-2-amine

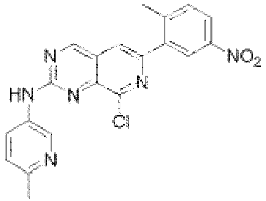

6-(2-methyl-5-nitrophenyl)-2-((6-methylpyridin-3-yl)amino)pyrido[3,4-*d*]pyrimidin-8(7*H*)-one (400 mg, 1 eq.) was placed in a round bottom flask, followed by the addition of phosphoryl chloride (40 eq.). The mixture was stirred at 70°C for 1 hour. After completion of the reaction, the reaction solution was diluted with 10% methanol/dichloromethane and washed with a saturated solution of sodium bicarbonate and then washed with brine. The organic layer was dried with magnesium sulfate and concentrated. The concentrate was purified by chromatography (0-10% methanol/dichloromethane) to obtain the title compound (250 mg, 60% yield) that was yellow in color. ¹H-NMR: (400 MHz, DMSO-d⁶): δ (ppm): 10.71 (1H, s), 9.57 (1H, s), 9.19 (1H, brs), 8.42 (1H, d, *J* = 8.4 Hz), 8.40 (1H, s), 8.36 (1H, d, *J* = 8.5 Hz), 8.35 (1H, s), 7.65 (1H, d, *J* = 8.5 Hz), 7.35 (1H, d, *J* = 8.4 Hz), 2.56 (3H, s), 2.48 (3H, s). LCMS (ESI): 407 (M + H)⁺.

### step 11: 6-(5-amino-2-methylphenyl)-8-chloro-N-(6-methylpyrimidin-3-yl)pyrido[3,4-d]pyrimidine-2-amine

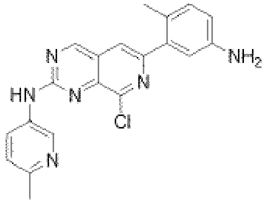

8-chloro-6-(2-methyl-5-nitrophenyl)-*N*-(6-methylpyridin-3-yl)pyrido[3,4-*d*]pyrimidine-2-amine (240 mg, 1 eq.), iron (330 mg, 10 eq.), ammonium chloride (313 mg, 10 eq.) and tetrahydrofuran: methanol: water (2:1:1, 6 mL) were placed in a round bottom flask. The mixture was stirred at 80°C for 1 hour. After completion of the reaction, the reaction solution was filtered through celite. The filtrate was washed with 20% methanol/dichloromethane and washed with a saturated aqueous solution of sodium bicarbonate and then washed with brine. The organic layer was dried with magnesium sulfate and concentrated. The concentrate was purified by chromatography (0-10% methanol/dichloromethane) to obtain the title compound (160 mg, 72% yield) that was yellow in color. ¹H-NMR: (400 MHz, DMSO-*d*₆) : δ (ppm) : 10.55 (1H, s), 9.54 (1H,s), 9.13 (1H, brs), 8.42 (1H, d, *J* = 8.4 Hz), 7.94 (1H, s), 7.28 (1H, d, *J* = 8.5 Hz), 6.98 (1H, d, *J* = 8.5 Hz), 6.76 (1H, s), 6.56 (1H, d, *J* = 8.4 Hz), 5.03 (2H, brs), 2.45 (3H, s), 2.23(3H, s). LCMS (ESI) : 377 (M + H)⁺.

### step 12: N-(3-(8-chloro-2-((6-methylpyridin-3-yl)amino)pyrido[3,4-d]pyrimidin-6-yl)-4-methylphenyl)-3-(trifluoromethyl)benzamide

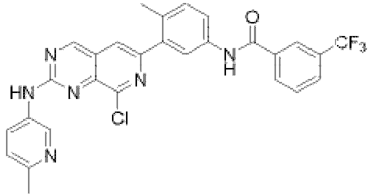

6-(5-amino-2-methylphenyl)-8-chloro-*N*-(6-methylpyrimidin-3-yl)pyrido[3,4-*d*]pyrimidine-2-amine (160 mg, 1 eq.) was placed in a round bottom flask, added and dissolved by the addition of tetrahydrofuran: dichloromethane (4:1, 5 mL). At 0°C, diisopropylethylamine was added thereto, followed by stirring for 10 minutes. Then, 3-trifluoromethyl benzoic chloride (132 mg, 1.5 eq.) was slowly added thereto dropwise. The reaction solution was stirred at room temperature for 3 hours. After completion of the reaction, the reaction solution was diluted with ethyl acetate, washed with a saturated aqueous solution of sodium bicarbonate and then washed with brine. The organic layer was dried with magnesium sulfate and concentrated. The concentrate was purification by chromatography (0-10% methanol/dichloromethane) to obtain the title compound (110 mg) that was yellow in color.

¹H-NMR: (400 MHz, CD₃OD): δ (ppm): 9.54 (1H, s), 9.16 (1H, s), 9.14 (1H, s), 8.42 (1H, d, *J* = 8.4 Hz), 8.04 (3H, m), 7.99 (1H, s), 7.94 (1H, s), 7.28 (1H, d, *J* = 8.5 Hz), 7.35(1H, d, *J* = 8.5 Hz), 7.30 (1H, d, *J* = 8.4 Hz), 2.50 (3H, s), 2.45 (3H, s). LCMS (ESI): 549 (M + H)⁺.

### Synthesis Example 2. Production of N-(4-methyl-3-(2-((6-methylpyridin-3-yl)amino)-8-vinylpyrido[3,4-d]pyrimidin-6-yl)phenyl)-3-(trifluoromethyl)benzamide (Compound No. 2)

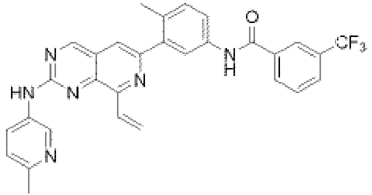

*N*-(3-(8-chloro-2-((6-methylpyridin-3-yl)amino)pyrido[3,4-d]pyrimidin-6-yl)-4-methylphenyl)-3-(trifluoromethyl)benzamide (Compound No. 1) (40 mg, 1 eq.), tributyl(vinyl)tin (32 mg, 1.3 eq.), Pd(PPh₃)₄ (2.8 mg, 0.03 eq.) and 1,4-dioxane (6 mL) were placed in a round bottom flask. The mixture was refluxed for 2 hours and cooled. After completion of the reaction, the reaction solution was diluted with ethyl acetate and then washed with brine. The organic layer was dried with magnesium sulfate and concentrated. The concentrate was purified by chromatography (0-10% methanol/dichloromethane) to obtain the title compound (12 mg) that was yellow in color.

¹H-NMR: (400 MHz, CDCl₃): δ (ppm) : 9.18 (1H, s), 9.56 (1H, s), 8.21 (2H, m), 8.15 (1H, d, *J* = 8.4 Hz), 8.0 (1H, d, J = 8.5 Hz), 7.99 (2H, m), 7.88 (1H, d, J = 8.4 Hz), 7.60 (3H,m), 7.45 (1H, d, J = 8.5 Hz), 7.32 (1H, d, J = 8.5 Hz), 6.78 (1H, d, J = 8.7 Hz), 5.74 (1H, m), 2.59 (3H, s), 2.48 (3H, s). LCMS (ESI): 541 (M + H)⁺.

### Synthesis Example 3. Production of N-(3-(8-ethyl-2-((6-methylpyridin-3-yl)amino)pyrido[3,4-d]pyrimidin-6-yl)-4-methylphenyl)-3-(trifluoromethyl)benzamide (Compound No. 3)

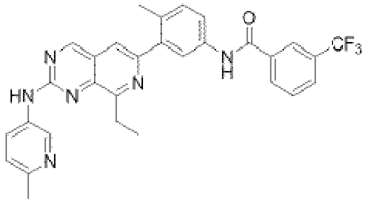

*N*-(4-methyl-3-(2-((6-methylpyridin-3-yl) amino) -8-vinylpyrido[3,4-*d*]pyrimidin-6-yl)phenyl)-3-(trifluoromethyl)benzamide (Compound No. 2) (10 mg, 1 eq.) was placed in a round bottom flask and dissolved by the addition of methanol (2 mL). Pd/C (3 mg, 30 wt%) was added thereto, and the atmosphere was replaced with hydrogen using a hydrogen balloon. After completion of the reaction, the reaction solution was filtered through celite. The filtrate was concentrated under reduced pressure. The concentrate was recrystallized from ethyl ether and hexane, filtered, and dried to obtain the title compound (2.5 mg) that was yellow in color.

¹H-NMR: (400 MHz, CDCl₃): δ (ppm) : 9.18 (1H, s), 8.96 (1H, s), 8.29 (1H, d), 8.26 (1H, s), 8.15 (1H, d), 7.90 (1H, s), 7.83 (2H, m), 7.66 (3H, m), 7.44 (1H, s), 7.35 (1H, d), 7.33(1H, d), 3.46 (2H, q), 2.59 (3H, s), 2.44 (3H, s), 1.5 (3H, t). LCMS (ESI): 543 (M + H)⁺.

### Synthesis Example 4. Production of N-(4-methyl-3-(2-((6-methylpyridin-3-yl)amino)pyrido[3,4-d]pyrimidin-6-yl)phenyl)-3-(trifluoromethyl)benzamide (Compound No. 4)

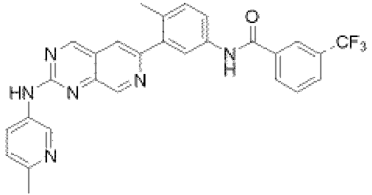

*N*-(3-(8-chloro-2-((6-methylpyridin-3-yl)amino)pyrido[3,4-d]pyrimidin-6-yl)-4-methylphenyl)-3-(trifluoromethyl)benzamide (Compound No. 1) (60 mg, 1 eq.) and tetrahydrofuran (5 mL) were placed in a round bottom flask, and the gas in the solvent was removed using a nitrogen balloon for 15 minutes. Next, PdCl₂(dppf) (8 mg, 0.1 eq.), tetramethylethylenediamine (44 mg, 3.4 eq.) and sodium borohydride (14 mg, 3.4 eq.) were added thereto dropwise at room temperature over 20 minutes. After completion of the reaction, the reaction solution was filtered through celite. The filtrate was diluted with ethyl acetate and then washed with brine. The organic layer was dried with magnesium sulfate and concentrated. The concentrate was purified by chromatography (0-10% methanol/dichloromethane) to obtain the title compound (10 mg, 17% yield) that was pale yellow in color.

¹H-NMR: (400 MHz, DMSO-*d*₆): δ (ppm): 10.54 (1H, s), 10.30 (1H, s), 9.54 (1H, s), 9.21(1H, s), 8.99 (1H, brs), 8.31 (3H, m), 7.98 (3H, m), 7.79 (2H, d, *J* = 8.4 Hz), 7.28 (1H,d, *J* = 8.5 Hz), 7.25 (1H, d, *J* = 8.5 Hz), 2.45 (3H, s), 2.38 (3H, s). LCMS (ESI): 515 (M + H)⁺.

### Synthesis Example 5. Production of N-(3-(2-((2-hydroxyethyl)amino)-8-methoxypyrido[3,4-d]pyrimidin-6-yl)-4-methylphenyl)-3-(trifluoromethyl)benzamide (Compound No. 5)

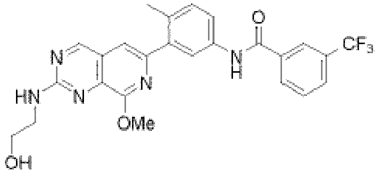

### step 1: 8-methoxy-6-(2-methyl-5-nitrophenyl)-2-(methylthio)pyrido[3,4-d]pyrimidine

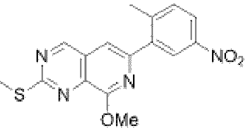

6-(2-methyl-5-nitrophenyl)-2-(methylthio) pyrido[3,4-*d*] pyrimidin-8-one (3.3 g, 1 eq.) produced in Step 7 of Synthesis Example 1, methyl iodide (2 mL, 3 eq.), potassium carbonate (5.5 g, 4 eq.) and acetonitrile (50 mL) were placed in a round bottom flask. The mixture was stirred at 80°C for 2 hours. After completion of the reaction, the reaction solution was filtered through celite and washed with 20% isopropanol/chloroform. The filtrate was concentrated under reduced pressure and purified by chromatography (0-5% methanol/dichloromethane) to obtain the title compound (0.54 g, 25% yield) that was yellow in color. LCMS (ESI): 343 (M +H)⁺.

### Step 2: 3-(8-methoxy-2-(methylthio)pyrido[3,4-d]pyrimidin-6-yl)-4-methylaniline

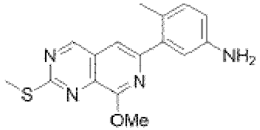

8-methoxy-6-(2-methyl-5-nitrophenyl)-2-(methylthio)pyrido[3,4-*d*]pyrimidine (300 mg, 1 eq.), iron (500 mg, 10 eq.), tetrahydrofuran: methanol (2:1, 15 mL) and an aqueous ammonium chloride solution (5 mL) were placed in a round bottom flask. The mixture was stirred at 80°C for 1 hour. After completion of the reaction, the reaction solution was filtered through celite and washed with 20% isopropanol/chloroform. The filtrate was washed with a saturated aqueous solution of sodium bicarbonate and then washed with brine. The organic layer was dried with magnesium sulfate and concentrated. The concentrate was recrystallized from ethyl ether, filtered, and dried to obtain the title compound (164 mg, 60% yield) that was yellow in color. LCMS (ESI): 313 (M + H)⁺.

### Step 3: N-(3-(8-methoxy-2-(methylthio)pyrimido[3,4-d]pyrimidin-6-yl)-4-methylphenyl)-3-(trifluoromethyl)benzamide

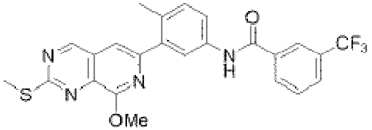

3-(8-methoxy-2-(methylthio)pyrido[3,4-*d*]pyrimidin-6-yl)-4-methylaniline (100 mg, 1 eq.), diisopropylethylamine (0.2 mL, 3 eq.) and tetrahydrofuran: dichloromethane (4:1, 15 mL) were placed in a round bottom flask. At 0 °C, 3-trifluoromethyl benzoic chloride (0.06 ml, 1.5 eq.) was added thereto, followed by stirring at room temperature for 2 hours. After completion of the reaction, the reaction solution was diluted with dichloromethane and washed with a saturated aqueous solution of sodium bicarbonate and then washed with brine. The organic layer was dried with magnesium sulfate and concentrated. The concentrate was recrystallized from ethyl ether, filtered, and dried to obtain the title compound (110 mg, 76% yield) that was yellow in color. LCMS (ESI): 485 (M + H)⁺.

### step 4: N-(3-(8-methoxy-2-(methylsulfonyl)pyrido[3,4-d]pyrimidin-6-yl)-4-methylphenyl)-3-(trifluoromethyl)benzamide

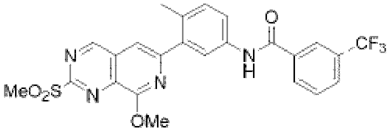

*N*-(3-(8-methoxy-2-(methylthio)pyrimido[3,4-d]pyrimidin-6-yl)-4-methylphenyl)-3-(trifluoromethyl)benzamide (100 mg, 1 eq.) and dichloromethane (10 mL) were placed in a round bottom flask. At 0°C, 3-chloroperbenzoic acid (70 mg, 2.5 eq.) was added thereto, followed by stirring at room temperature for 1 hour. After completion of the reaction, the reaction solution was diluted with 20% isopropanol/chloroform and washed with a saturated aqueous solution of sodium bicarbonate and then washed with brine. The organic layer was dried with magnesium sulfate and concentrated. The concentrate was recrystallized from ethyl ether, filtered, and dried to obtain the title compound (52 mg, 49% yield) that was yellow in color. LCMS (ESI): 517 (M + H)⁺.

### step 5: N-(3-(2-((2-hydroxyethyl)amino)-8-methoxypyrido[3,4-d]pyrimidin-6-yl)-4-methylphenyl)-3-(trifluoromethyl)benzamide

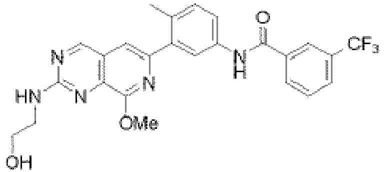

*N*-(3-(8-methoxy-2-(methylsulfonyl)pyrido[3,4-d]pyrimidin-6-yl)-4-methylphenyl)-3-(trifluoromethyl)benzamide (50 mg, 1 eq.), 2-aminoethanol (2 eq.) and dimethylformamide (2 mL) were placed in a round bottom flask. The mixture was stirred at 80°C for 2 hours. After completion of the reaction, the reaction solution was concentrated under reduced pressure and purified by chromatography to obtain the title compound that was yellow in color.

LCMS (ESI): 498 (M + H)⁻.

### Synthesis Example 6. Production of N-(3-(2-(cyclopropylamino)-8-methoxypyrido[3,4-d]pyrimidin-6-yl)-4-methylphenyl)-3-(trifluoromethyl)benzamide (Compound No. 6)

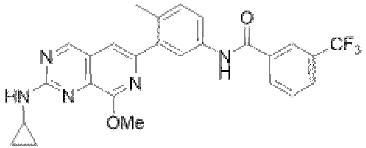

The experiment was conducted in the same manner as in Synthesis Example 5 (the production of Compound No. 5), except that cyclopropylamine was used instead of 2-aminoethanol in step 5.

LCMS (ESI): 494 (M + H)+.

### Synthesis Example 7. Production of N-(3-(8-methoxy-2-morpholinopyrido[3,4-d]pyrimidin-6-yl)-4-methylphenyl)-3-(trifluoromethyl)benzamide (Compound No. 7)

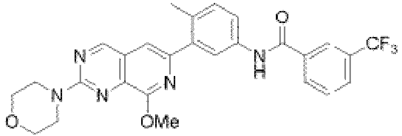

The experiment was conducted in the same manner as in Synthesis Example 5 (the production of Compound No. 5), except that morpholine was used instead of 2-aminoethanol in step 5.

LCMS (ESI): 524 (M + H)+.

### Synthesis Example 8. Production of N-(3-(8-methoxy-2-((6-methylpyridin-3-yl)amino)pyrido[3,4-d]pyrimidin-6-yl)-4-methylphenyl)-3-(trifluoromethyl)benzamide (Compound No. 8)

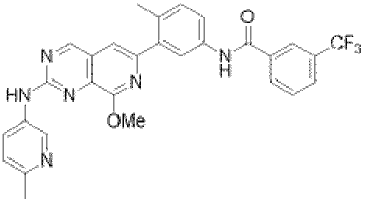

*N*-(3-(8-methoxy-2-(methylsulfonyl)pyrido[3,4-*d*]pyrimidin-6-yl)-4-methylphenyl)-3-(trifluoromethyl)benzamide (50 mg, 1 eq.) produced in step 4 of Synthesis Example 5 (the production of Compound No. 5), *N*-(6-methylpyridin-3-yl)formamide (2 eq.), cesium carbonate (3 eq.) and dimethylformamide (2 mL) were placed in a round bottom flask. The mixture was stirred at 80°C for 2 hours. After completion of the reaction, the reaction solution was concentrated under reduced pressure and purified by chromatography to obtain the tittle compound that was yellow in color.

LCMS (ESI): 545 (M + H)⁻.

### Synthesis Example 9. Production of N-(3-(8-methoxy-2-((6-morpholinopyridin-3-yl)amino)pyrido[3,4-d]pyrimidin-6-yl)-4-methylphenyl)-3-(trifluoromethyl)benzamide (Compound No. 9)

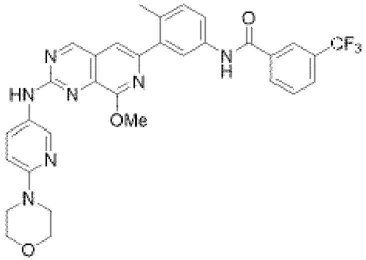

The experiment was conducted in the same manner as in Synthesis Example 8 (the production of Compound No. 8), except that *N*-(6-morpholinopyridin-3-yl)formamide was used instead of *N*-(6-methylpyridin-3-yl)formamide.

LCMS (ESI): 616 (M + H)⁻.

### Synthesis Example 10. Production of N-(3-(8-methoxy-2-((2-methoxy-4-morpholinophenyl)amino)pyrido[3,4-d]pyrimidin-6-yl)-4-methylphenyl)-3-(trifluoromethyl)benzamide (Compound No. 10)

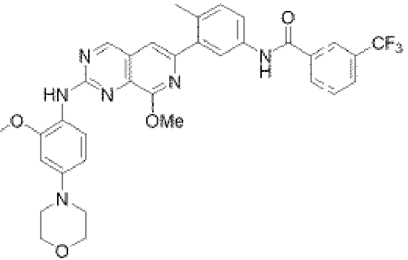

The experiment was conducted in the same manner as in Synthesis Example 8 (the production of Compound No. 8), except that *N*-(2-methoxy-4-morpholinophenyl)formamide was used instead of *N*-(6-methylpyridin-3-yl)formamide.

LCMS (ESI): 645 (M + H)⁻.

### Synthesis Example 11. Production of N-(3-(2-((4-(4-ethylpiperazin-1-yl)phenyl)amino)-8-methoxypyrido[3,4-d]pyrimidin-6-yl)-4-methylphenyl)-3-(trifluoromethyl)benzamide (Compound No. 11)

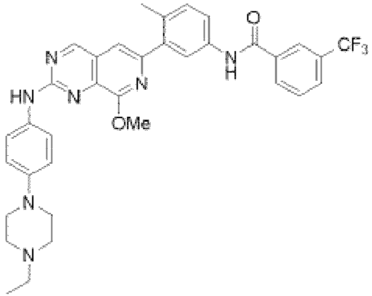

The experiment was conducted in the same manner as in Synthesis Example 8 (the production of Compound No. 8), except that N-(4-(4-ethylpiperazin-1-yl)phenyl)formamide was used instead of N-(6-methylpyridin-3-yl)formamide.

LCMS (ESI): 642 (M + H)⁻.

### Synthesis Example 12. Production of N-(4-methyl-3-(2-((6-methylpyridin-3-yl)amino)pyrido[3,4-d]pyrimidin-6-yl)phenyl)-4-(morpholinemethyl)-3-(trifluoromethyl)benzamide (Compound No. 12)

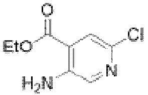

### Step 1: Ethyl-5-amino-2-chloroisonicotinate

Ethyl-2-chloro-5-chloroisonicotinate (30 g, 129 mmol), Fe (72 g, 1290 mmol), NH₄Cl (66 g, 1290 mmol), and tetrahydrofuran: methanol: water (2:1:1) (450 ml) were placed in a round bottom flask and stirred at 80°C for 1 hour. After completion of the reaction, the reaction solution was filtered through celite. The filtrate was diluted with ethyl acetate, and then washed with brine. The organic layer was dried with magnesium sulfate and concentrated. Recrystallization from hexane gave the title compound (24.5 g) that was brown in color. ¹H-NMR: (400 MHz, DMSO-*d*₆) : δ (ppm): 8.06 (1H, s), 7.49 (1H, s), 6.78(2H, brs), 4.30 (2H, q), 1.31 (3H, t). LCMS (ESI): 201 (M + H)⁺.

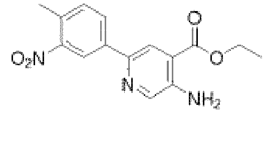

### Step 2: Ethyl-5-amino-2-(4-methyl-3-nitrophenyl)isonicotinate

5-amino-2-chloroisonicotinate (24.5 g, 122.2 mmol), 4,4,5,5-tetramethyl-2-(2-methyl-5-nitrophenyl)-1,3,2-dioxaborane (38.6 g, 146.6 mmol), Na₂CO₃ (25.8 g, 244.3 mmol), and dioxane/water (4:1) (611 ml) were placed in a round bottom flask, followed by degassing for 20 minutes. Next, Pd(PPh₃)₄ (7.05 g, 6.11 mmol) was added thereto, followed by stirring 110°C for 12 hours. After completion of the reaction, the reaction solution was filtered through celite. The filtrate was diluted with ethyl acetate and then washed with brine. The organic layer was dried with magnesium sulfate and concentrated. Recrystallization from ether gave the title compound (15 g) that was yellow in color. ¹H-NMR: (400 MHz, DMSO-*d*₆) : δ (ppm): 8.38 (1H, s), 8.16 (1H, s), 8.12 (1H, d), 7.70 (1H, s), 7.56 (1H, d), 6.91(2H, brs), 4.32 (2H, q), 2.44 (3H, s), 1.32 (3H, t). LCMS (ESI): 302 (M + H)⁺.

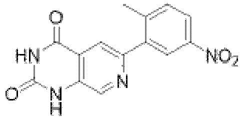

### Step 3: 6-(2-methyl-5-nitrophenyl)pyrido[3,4-d]pyrimidin-2,4(1H,3H)-dione

5-amino-2-(4-methyl-3-nitrophenyl)isonicotinate (15 g, 49.8 mmol) and urea (60 g, 996 mmol) were placed in a round bottom flask and heated at 180°C. After completion of the reaction, water was added to the reaction mixture which was then refluxed for 30 minutes and cooled. The resulting solid was filtered using water and dried to obtain the title compound (13.2 g) that was white in color. ¹H-NMR: (400 MHz, DMSO-*d*₆): δ(ppm): 11.66 (2H, brs), 8.69 (1H, s), 8.23 (1H, s), 8.18 (1H, d), 7.96 (1H, s), 7.62 (1H, d), 6.91 (2H, brs), 2.47 (3H, s). LCMS (ESI): 299 (M + H)⁺.

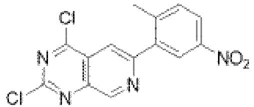

### Step 4: 2,4-dichloro-6-(2-methyl-5-nitrophenyl)pyrido[3,4-d]pyrimidine

6-(2-methyl-5-nitrophenyl)pyrido[3,4-*O*]pyrimidin-2,4(1*H*, 3*H*)-dione (13 g, 43.6 mmol), diethylaniline (26 g, 174.5 mmol) and POCl₃ were placed in a round bottom flask, refluxed and cooled. After completion of the reaction, POCl₃ was removed by drying under reduced pressure, and then the reaction product was added to ice water. The resulting solution was filtered and washed with ethyl acetate and ether. The solid was dried to obtain the title compound (6.5 g) that was bright red in color. ¹H-NMR: (400 MHz, CDCl₃): δ (ppm): 9.58 (1H, s), 8.41 (1H, s), 8.25 (1H, d), 8.15 (1H, s), 7.53 (1H, d), 2.54 (3H, s). LCMS (ESI): 335(M + H)⁺.

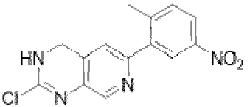

**step 5:** 2,4-dichloro-6-(2-methyl-5-nitrophenyl)pyrido[3,4-*d*]pyrimidine (6.5 g, 19.5 mmol), and tetrahydrofuran/ethanol (2:1) (450 ml) were placed in a round bottom flask. At 0°C, NaBH₄ (7.4 g, 195 mmol) was added slowly thereto, followed by stirring at room temperature for 30 minutes. After completion of the reaction, ice water and ethyl acetate were added. The reaction solution was neutralized with NH₄Cl. The organic layer was washed with brine and then dried with magnesium sulfate and concentrated. Recrystallization from ether gave the title compound (4.5 g) that was yellow in color. ¹H-NMR: (400 MHz, CDCl₃) : δ (ppm) : 8.15 (3H, m), 7.59 (1H, d), 7.34 (1H, s), 4.74 (2H, s), 2.46 (3H, s). LCMS (ESI): 303 (M + H)+.

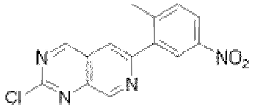

### Step 6: 2-chloro-6-(2-methyl-5-nitrophenyl)pyrido[3,4-d]pyrimidine

2-chloro-6-(2-methyl-5-nitrophenyl)-3,4-dihydropyrido[3,4-d]pyrimidine (4.5 g, 14.9 mmol), and dichloromethane (100 ml) were placed in a round bottom flask. At 0°C, DDQ (10 g, 44.7 mmol) was slowly added thereto, followed by stirring at room temperature for 24 hours. After completion of the reaction, the reaction solution was filtered using dichloromethane and dried under reduced pressure. The solid formed by adding methanol was filtered to obtain the title compound (2.4 g) that was white in color. ¹H-NMR: (400 MHz, CDCl₃): δ (ppm): 9.84 (1H, s), 9.59 (1H, s), 8.46 (1H, s), 8.39 (1H, s), 8.26 (1H, d),8.70 (1H, d), 2.54 (3H, s). LCMS (ESI): 301 (M + H)⁺.

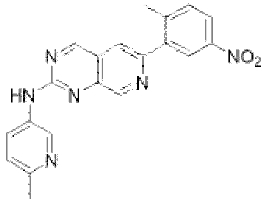

### step 7: 6-(2-methyl-5-nitrophenyl)-N-(6-methylpyridin-3-yl)pyrido[3,4-d]pyrimidin-2-amine

2-chloro-6-(2-methyl-5-nitrophenyl)pyrido[3,4-*d*]pyrimidine (2.4 g, 8 mmol), 6-methylpyridine-3-amine (1.3 g, 12 mmol), Xphos (0.95 g, 2 mmol), and K₂CO₃ (3.3 g, 24 mmol) were placed in a round bottom flask, followed by degassing for 20 minutes. Next, Pd₂(dba)₃ (1.8 g, 2 mmol) was added to the mixture which was then refluxed for 12 hours and cooled. After completion of the reaction, water and ethyl acetate were added to the reaction solution, and the organic layer was washed with brine. The organic layer was dried with magnesium sulfate and concentrated. Purification by chromatography (methanol/dichloromethane) gave the title compound (630 mg) that was yellow in color. ¹H-NMR: (400 MHz, CDCl₃): δ (ppm) : 10.35 (1H, s), 9.53 (1H, s), 9.22 (1H, s), 8.98 (1H, s), 8.38 (1H, s), 8.34 (1H, d), 8.23 (1H, d), 8.14 (1H, s),7.66 (1H, d), 7.26 (1H, d), 2.54 (3H, s), 2.45 (3H, s). LCMS (ESI): 373 (M + H)⁺.

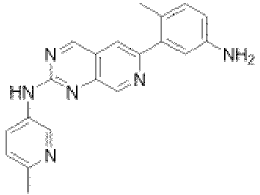

### step 8: 6-(5-amino-2-methylphenyl)-N-(6-methylpyridin-3-yl)pyrido[3,4-d]pyrimidine-2-amine

6-(2-methyl-5-nitrophenyl)-*N*-(6-methylpyridin-3-yl)pyrido[3,4-*d*]pyrimidin-2-amine (630 mg, 1.7 mmol), ethanol and SnCl₂ (1.98 g, 8.5 mmol) were placed in a round bottom flask, refluxed for 3 hours and cooled. After completion of the reaction, chloroform/isopropyl alcohol and a sodium bicarbonate aqueous solution were added thereto. The organic layer was washed with brine, and then the organic layer was dried with magnesium sulfate and concentrated. Purification by chromatography (methanol/dichloromethane) gave the title compound (410 mg) that was yellow in color. ¹H-NMR: (400 MHz, DMSO-*d*₆): δ (ppm) : 10.26 (1H, s), 9.51 (1H, s), 9.16 (1H, s), 8.98 (1H, s), 8.31 (1H, d), 7.87 (1H, s), 7.25 (1H, d), 6.96 (1H, d), 6.80 (1H, s), 6.55 (1H, d), 4.97 (2H, brs), 2.54 (3H, s), 2.45 (3H, s). LCMS (ESI): 343 (M + H) +.

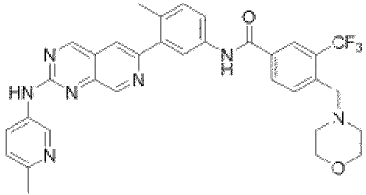

### Step 9: N-(4-methyl-3-(2-((6-methylpyridin-3-yl)amino)pyrido[3,4-d]pyrimidin-6-yl)phenyl)-4-(morpholinemethyl)-3-(trifluoromethyl)benzamide

6-(5-amino-2-methylphenyl)-*N*-(6-methylpyridin-3-yl)pyrido[3,4-d]pyrimidine-2-amine (410 mg, 1.16 mmol), tetrahydrofuran, and DIPEA (1.1 ml, 6.9 mmol) were placed in a round bottom flask. 4-(morpholinomethyl)-3-(trifluoromethyl)benzoyl chloride (703 mg, 2.9 mmol) was added thereto at 0°C, followed by stirring at room temperature for 1 hour. After completion of the reaction, ethyl acetate and water were added to the reaction solution. The organic layer was washed with brine, and then the organic layer was dried with magnesium sulfate and concentrated. After concentration, the concentrate was recrystallized from ethyl acetate and filtered to obtain the title compound (290 mg) that was yellow in color.

¹H-NMR: (400 MHz, DMSO-*d*₆) : δ (ppm) : 9.82 (1H, s), 9.48 (1H, s), 9.29 (1H, s), 9.24 (1H, s), 9.07 (1H, s), 8.48 (1H, d), 8.34 (1H, s), 8.29 (1H, d), 8.05 (2H, m), 7.96 (1H, s),7.85 (1H, d), 7.34 (1H, d), 7.28 (1H, d), 3.75 (2H, s), 3.67 (4H, m), 2.54 (4H, m), 2.51 (3H, s), 2.44 (3H, s). LCMS (ESI): 614 (M + H)⁺.

### Synthesis Example 13. Production of 3-(4-methyl-1H-imidazol-1-yl)-N-(4-methyl-3-(2-((6-methylpyridin-3-yl)amino)pyrido[3,4-d]pyrimidin-6-yl)phenyl)-5-(trifluoromethyl)benzamide (Compound No. 13)

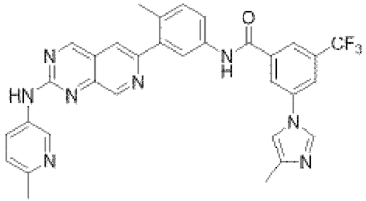

The experiment was conducted in the same manner as in Synthesis Example 12 (the production of Compound No. 12), except that 3-(4-methyl-1*H*-imidazo-1-yl)-5-(trifluoromethyl)benzoyl chloride was used instead of 4-(morpholinomethyl)-3-(trifluoromethyl)benzoyl chloride.

LCMS (ESI): 595 (M + H)⁻.

### Synthesis Example 14. Production of N-(4-methyl-3-(2-((6-methylpyridin-3-yl)amino)pyrido[3,4-d]pyrimidin-6-yl)phenyl)-3-morpholino-5-(trifluoromethyl)benzamide (Compound No. 14)

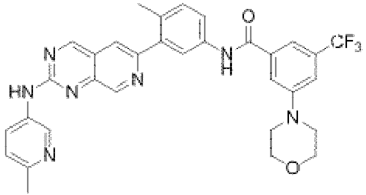

The experiment was conducted in the same manner as in Synthesis Example 12 (the production of Compound No. 12), except that 4-morpholino-3-(trifluoromethyl)benzoyl chloride was used instead of 4-(morpholinomethyl)-3-(trifluoromethyl)benzoyl chloride.

¹H-NMR: (400 MHz, DMSO-*d*₆) : δ (ppm) : 9.77 (1H, s), 9.46 (1H, s), 9.29 (1H, s), 9.23 (1H, s), 9.07 (1H, s), 8.47 (1H, d), 8.00 (1H, s), 7.94 (1H, s), 7.82 (2H, m), 7.73 (1H, s) , 7.40 (1H, s), 7.32 (1H, d), 7.27 (1H, d), 3.83 (4H, m), 3.45 (4H, m), 2.50 (3H, s), 2.43 (3H, s). LCMS (ESI): 600 (M + H)⁺.

### Synthesis Example 15. Production of 4-(2,4-dimethyl-1H-imidazo-1-yl)-N-(4-methyl-3-(2-((6-methylpyridin-3-yl)amino)pyrido[3,4-d]pyrimidin-6-yl)phenyl)-3-(trifluoromethyl)benzamide (Compound No. 15)

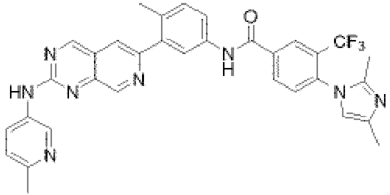

The experiment was conducted in the same manner as in Synthesis Example 12 (the production of Compound No. 12), except that 3-(2,4-dimethyl-1*H*-imidazo-1-yl)-5-(trifluoromethyl)benzoyl chloride was used instead of 4-(morpholinomethyl)-3-(trifluoromethyl)benzoyl chloride.

¹H-NMR: (400 MHz, DMSO-*d*₆) : δ (ppm) : 9.99 (1H, s), 9.48 (1H, s), 9.30 (1H, s), 9.24 (1H, s), 9.07 (1H, s), 8.53 (1H, s), 8.47 (2H, m), 8.05 (1H, s), 7.96 (1H, s), 7.86 (1H, d), 7.69 (1H, d), 7.36 (1H, d), 7.27 (1H, d), 6.86 (1H, s), 2.50 (3H, s), 2.44 (3H, s), 2.15 (3H, s), 2.08 (3H, s). LCMS (ESI): 609 (M + H)⁺.

### Synthesis Example 16. Production of 4-(4-hydroxypiperidin-1-yl)-N-(4-methyl-3-(2-((6-methylpyridin-3-yl)amino)pyrido[3,4-d]pyrimidin-6-yl)phenyl)-3-(trifluoromethyl)benzamide (Compound No. 16)

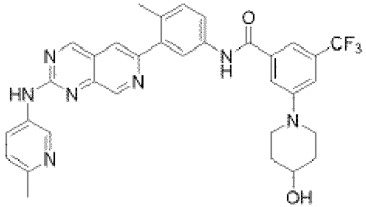

The experiment was conducted in the same manner as in Synthesis Example 12 (the production of Compound No. 12), except that 4-(4-hydroxypiperidin-1-yl)-3-(trifluoromethyl)benzoyl chloride was used instead of 4-(morpholinomethyl)-3-(trifluoromethyl)benzoyl chloride.

LCMS (ESI): 614 (M + H)⁻.

### Synthesis Example 17. Production of N-(3-(2-aminopyrido[3,4-d]pyrimidin-6-yl)-4-methylphenyl)-3-(trifluoromethyl)benzamide (Compound No. 17)

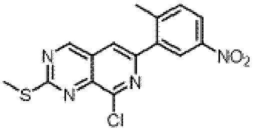

### Step 1: Ethyl-5-amino-2-chloroisonicotinate

8-chloro-6-(2-methyl-5-nitrophenyl)-2-(methylthio)pyrido[3,4-*d*]pyrimidine (2 g) produced in Step 7 of Synthesis Example 1, and POCl₃ (40 mL) were added and stirred at 70°C. After completion of the reaction, POCl₃ was removed by drying under reduced pressure, and the reaction product was added to ice water. The resulting solid was filtered using ether to obtain the title compound (1.4 g) that was brown in color. ¹H-NMR: (400 MHz, acetone-*d*₆) : δ (ppm) : 9.59 (1H, s), 8.44 (1H, s), 8.43 (1H, s), 8.27 (1H, d, *J* = 8.5 Hz), 7.68 (1H, d, *J* = 8.5 Hz), 2.76 (3H, s), 2.62 (3H, s). LCMS (ESI): 347 (M + H)⁺.

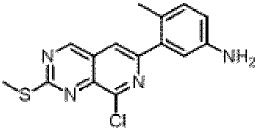

### step 2: 3-(8-chloro-2-(methylthio)pyrido[3,4-d]pyrimidin-6-yl)-4-methylaniline

8-chloro-6-(2-methyl-5-nitrophenyl)-2-(methylthiol)pyrido[3,4-d]pyrimidine (1.4 g, 1 eq.), tetrahydrofuran: methanol: water (2:1:1, 20 mL), Fe (2.2 g, 10 eq.), and NH₄Cl (2.0 g, 10 eq.) were placed in a round bottom flask, followed by stirring at 80°C for 1 hour. After completion of the reaction, the reaction solution was filtered through celite. The filtrate was diluted with ethyl acetate and then washed with brine. The organic layer was dried with magnesium sulfate and concentrated to obtain the title compound (800 mg) that was yellow in color. ¹H-NMR: (400 MHz, DMSO-*d*₆): δ (ppm): 9.61 (1H, s), 8.07 (1H, s), 6.99(1H, d, J = 8.5 Hz), 6.78 (1H, s), 6.59 (1H, d, J = 8.5 Hz), 5.06 (2H, brs) 2.70 (3H, s), 2.22 (3H, s). LCMS (ESI): 317 (M + H)⁺.

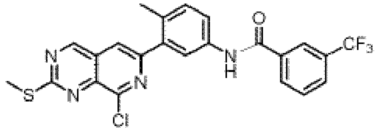

### Step 3: N-(3-(8-chloro-2-(methylthiol)pyrido [3,4-d]pyrimidin-6-yl)-4-methyl)-3-(trifluoromethyl)benzamide

3-(8-chloro-2-(methylthio)pyrido[3,4-*d*]pyrimidin-6-yl)-4-methylaniline (800 mg, 1 eq.), tetrahydrofuran (15 mL), and DIPEA (1.3 mL, 3 eq.) were placed in a round bottom flask. At 0°C, 3-trifluoromethyl benzoyl chloride (1.05 g, 2 eq.) was added slowly thereto, followed by stirring at room temperature for 2 hours. After completion of the reaction, dichloromethane and a sodium bicarbonate solution were added to the reaction solution. The organic layer was washed with brine, and then the organic layer was dried with magnesium sulfate and concentrated. After concentration, the concentrate was recrystallized from ether and filtered to obtain the title compound (750 mg) that was yellow in color. ¹H-NMR: (400 MHz, DMSO-*d*₆): δ (ppm): 10.58 (1H, s), 9.64 (1H, s), 8.33 (1H, s), 8.28 (1H, d, *J* = 8.5 Hz), 8.19 (1H, s), 7.98 (2H, m) , 7.81 (3H, m), 7.38 (1H, d, *J* = 8.5 Hz), 2.701 (3H, s), 2.40 (3H, s). LCMS (ESI): 489 (M + H)⁺.

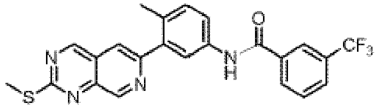

### Step 4: N-(4-methyl-3-(2-(methylthio)pyrido[3,4-d]pyrimidin-6-yl)phenyl)-3-(trifluoromethyl)benzamide

*N*-(3-(8-chloro-2-(methylthio)pyrido[3,4-*d*]pyrimidin-6-yl)-4-methylphenyl)-3-(trifluoromethyl)benzamide (750 mg, 1 eq.), Et3N (1.25 mL, 5.6 eq.), HCOOH (0.17 mL, 3.5 eq.), Pd(PPh₃)₄ (165 mg, 0.1 eq.), and dimethylsulfoxide were placed in a round bottom flask, followed by stirring 80°C for 2 hours. After completion of the reaction, ethyl acetate and water were added to the reaction solution. The organic layer was washed with brine, and then the organic layer was dried with magnesium sulfate and concentrated. After concentration, the concentrate was recrystallized from ether and filtered to obtain the title compound (400 mg) that was yellow in color. ¹H-NMR: (400 MHz, DMSO-*d*₆): δ (ppm): 10.55 (1H, s), 9.61 (1H, s), 9.40 (1H, s), 8.30 (2H, m), 8.15 (1H, s), 7.89 (2H, m), 7.79 (2H, m), 7.37 (1H, d, *J* = 8.5Hz), 2.71 (3H, s), 2.38 (3H, s). LCMS (ESI): 455 (M + H)⁺.

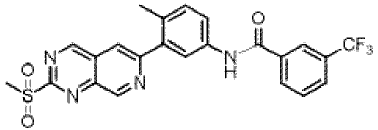

### Step 5: N-(4-methyl-3-(2-(methylsulfonyl) pyrido [3,4-d]pyrimidin-6-yl)phenyl)-3-(trifluoromethyl)benzamide

In a round bottom flask, *N*-(4-methyl-3-(2-(methylthio)pyrido[3,4-d]pyrimidin-6-yl)phenyl)-3-(trifluoromethyl)benzamide (400 mg, 1 eq.) was dissolved in dichloromethane (10 mL). At 0°C, 3-chloroperbenzoic acid (379 mg, 2.5 eq.) was added to the solution, followed by stirring at room temperature for 24 hours. After completion of the reaction, the reaction solution was diluted with 10% methanol/dichloromethane and washed with a saturated aqueous solution of sodium bicarbonate and then washed with brine. The organic layer was dried with magnesium sulfate and concentrated. After concentration, the concentrate was recrystallized from ether and filtered to obtain the title compound (300 mg) that was yellow in color. LCMS (ESI): 487 (M + H)⁺.

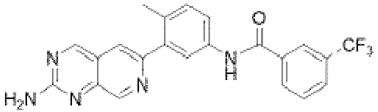

### Step 6: N-(3-(2-aminopyrido[3,4-d]pyrimidin-6-yl)-4-methylphenyl)-3-(trifluoromethyl)benzamide

The experiment was conducted in the same manner as in Step 5 of Synthesis Example 5, except that ammonia water was used instead of aminoethanol.

¹H-NMR: (400 MHz, DMSO-*d*₆) : δ (ppm) : 9.83 (1H, brs), 9.33 (1H, s), 9.01 (1H, s), 8.35 (1H, s), 8.34 (1H, d, *J* = 8.8 Hz), 8.00 (1H, s), 7.94 (1H, d, *J* = 7.6 Hz), 7.82 (3H, m),7.32 (1H, d, *J* = 8.4 Hz), 6.59 (2H, brs), 2.42 (3H, s). LCMS (ESI): 424 (M + H)⁺.

### Synthesis Example 18. Production of N-(3-(2-((2-hydroxyethyl)amino)pyrido[3,4-d]pyrimidin-6-yl)-4-methylphenyl)-3-(trifluoromethyl)benzamide (Compound No. 18)

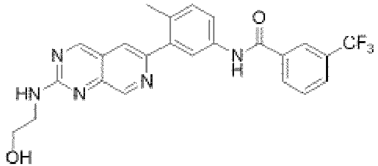

The experiment was conducted in the same manner as in Step 5 of Synthesis Example 5.

¹H-NMR: (400 MHz, DMSO-*d₆*): δ (ppm) : 10.51 (1H, s), 9.32 (1H, brs), 9.00 (1H, s), 8.33 (1H, s), 8.26 (1H, d, *J* = 7.6 Hz), 7.94 (2H, m), 7.86 (1H, s), 7.77 (3H, m), 7.33 (1H, d, *J* = 8.4 Hz), 4.76 (1H, brs), 3.57 (2H, m), 3.50 (2H, m), 2.90 (1H, m), 2.37 (3H, s). LCMS (ESI): 468 (M + H)⁺.

### Synthesis Example 19. Production of N-(3-(2-(cyclicpropylamino)-pyrido[3,4-d]pyrimidin-6-yl)-4-methylphenyl)-3-(trifluoromethyl)benzamide (Compound No. 19)

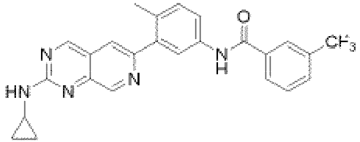

The experiment was conducted in the same manner as in Step 5 of Synthesis Example 5 (the production of Compound No. 5), except that cyclopropylamine was used instead of 2-aminoethanol.

¹H-NMR: (400 MHz, DMSO-*d₆*): δ (ppm) : 10.52 (1H, s), 9.32 (1H, brs), 9.06 (1H, s), 8.33 (1H, s), 8.28 (1H, d, *J* = 8.0 Hz), 8.04 (1H, s), 7.96 (2H, m), 7.94 (1H, s), 7.82 (2H, m),7.32 (1H, d, *J* = 8.4 Hz), 2.90 (1H, m), 2.37 (3H, s), 0.77 (2H, m), 0.58 (2H, m). LCMS (ESI): 464 (M + H)⁺.

### Synthesis Example 20. Production of N-(4-methyl-3-(2-(phenylamino)pyrido[3,4-d]pyrimidin-6-yl)phenyl)-3-(trifluoromethyl)benzamide (Compound No. 20)

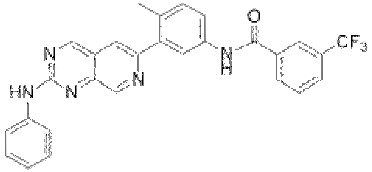

The experiment was conducted in the same manner as in Synthesis Example 8, except that N-phenylformamide was used instead of *N*-(6-methylpyridin-3-yl)formamide.

LCMS (ESI): 500 (M + H)⁻.

### Synthesis Example 21. Production of N-(4-methyl-3-(2-((1-methyl-1H-pyrazo-4-yl)amino)pyrido[3,4-d]pyrimidin-6-yl)phenyl)-3-(trifluoromethyl)benzamide (Compound No. 21)

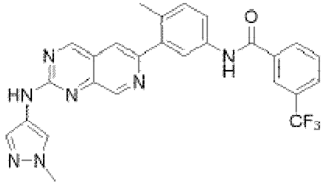

The experiment was conducted in the same manner as in Synthesis Example 8, except that *N*-(1-methyl-1*H*-pyrazol-4-yl)formamide was used instead of *N*-(6-methylpyridin-3-yl)formamide.

¹H-NMR: (400 MHz, acetone-*d*₆) : δ (ppm) : 9.83 (1H, s), 9.37 (1H, s), 9.22 (1H, s), 8.35 (3H, m), 8.02 (1H, s), 8.01 (1H, d), 7.93 (1H, s), 7.87 (2H, m), 7.76 (1H, s), 7.34 (1H,d), 3.93 (3H, s), 2.43 (3H, s). LCMS (ESI): 504 (M + H)⁺.

### Synthesis Example 22. Production of N-(3-(2-((1,3-dimethyl-1H-pyrazol-5-yl)amino)pyrido[3,4-d]pyrimidin-6-yl)-4-methylphenyl)-3-(trifluoromethyl)benzamide (Compound No. 22)

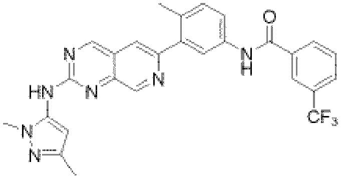

The experiment was conducted in the same manner as in Synthesis Example 8, except that *N*-(1,3-dimethyl-1*H*-pyrazol-5-yl)formamide was used instead of *N*-(6-methylpyridin-3-yl)formamide.

LCMS (ESI): 518 (M + H)⁻.

### Synthesis Example 23. Production of N-(4-methyl-3-(2-((4-methylpyridin-3-yl)amino)pyrido[3,4-d]pyrimidin-6-yl)phenyl)-3-(trifluoromethyl)benzamide (Compound No. 23)

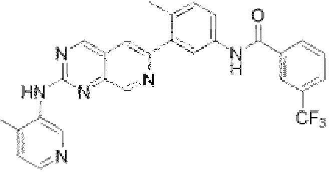

The experiment was conducted in the same manner as in Synthesis Example 8, except that *N*-(4-methylpyridin-3-yl)formamide was used instead of *N*-(6-methylpyridin-3-yl)formamide.

LCMS (ESI): 515 (M + H)⁻.

### Synthesis Example 24. Production of N-(3-(2-((2,6-dimethylpyridin-3-yl)amino)pyrido[3,4-d]pyrimidin-6-yl)-4-methylphenyl)-3-(trifluoromethyl)benzamide (Compound No. 24)

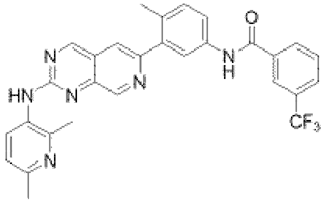

The experiment was conducted in the same manner as in Synthesis Example 8, except that *N*-(2,6-dimethylpyridin-3-yl)formamide was used instead of *N*-(6-methylpyridin-3-yl)formamide.

¹H-NMR: (400 MHz, acetone-*d*₆) : δ (ppm) : 9.84 (1H, s), 9.46 (1H, s), 9.08 (1H, s), 8.47 (1H, s), 8.36 (2H, m), 8.17 (1H, d), 8.03 (1H, s), 7.94 (2H, s), 7.82 (2H, m), 7.34 (1H,d), 7.16 (1H, d), 2.57 (3H, s), 2.50 (3H, s), 2.43 (3H, s). LCMS (ESI): 529 (M + H)⁺.

### Synthesis Example 25. Production of N-(3-(2-((4-(4-ethylpiperazin-1-yl)phenyl)amino)pyrido[3,4-d]pyrimidin-6-yl)-4-methylphenyl)-3-(trifluoromethyl)benzamide (Compound No. 25)

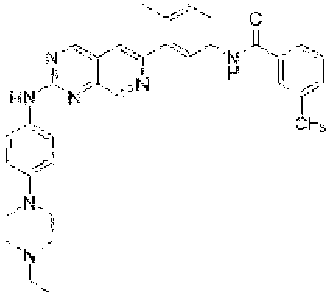

The experiment was conducted in the same manner as in Synthesis Example 8, except that *N*-(4-(4-ethylpiperazin-1-yl)phenyl)formamide was used instead of *N*-(6-methylpyridin-3-yl) formamide.

LCMS (ESI): 612 (M + H)⁻.

### Synthesis Example 26. Production of N-(3-(2-((3-(4-ethylpiperazin-1-yl)phenyl)amino)pyrido[3,4-d]pyrimidin-6-yl)-4-methylphenyl)-3-(trifluoromethyl)benzamide (Compound No. 26)

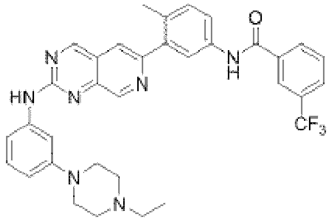

The experiment was conducted in the same manner as in Synthesis Example 8, except that *N*-(3-(4-ethylpiperazin-1-yl)phenyl)formamide was used instead of *N*-(6-methylpyridin-3-yl)formamide.

LCMS (ESI): 612 (M + H)⁻.

According to the methods of the Synthesis Examples, the compounds of Compound Nos. 1 to 26 were synthesized.

### Mode for Invention

Hereinafter, the present invention will be described in detail with reference to examples. However, the following examples are merely for illustrating the present invention, and the scope of the present invention is not limited by the following examples.

Based on Synthesis Examples 1 to 26 above, compounds of Examples 1 to 26 were produced. Examples 1 to 26 are as follows.

### [Examples]

### Example 1. (Compound No. 1) N-(3-(8-chloro-2-((6-methylpyridin-3-yl)amino)pyrido[3,4-d]pyrimidin-6-yl)-4-methylphenyl)-3-(trifluoromethyl)benzamide

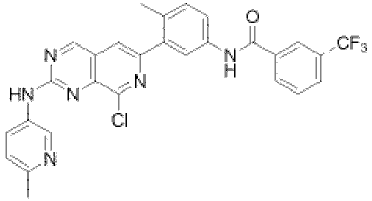

¹H-NMR: (400 MHz, CD₃OD): δ (ppm) : 9.54 (1H, s), 9.16 (1H, s), 9.14 (1H, s), 8.42 (1H, d, *J* = 8.4 Hz), 8.04 (3H, m), 7.99 (1H, s), 7.94 (1H, s), 7.28 (1H, d, *J* = 8.5 Hz), 7.35(1H, d, *J* = 8.5 Hz), 7.30 (1H, d, *J* = 8.4 Hz), 2.50 (3H, s), 2.45 (3H, s). LCMS (ESI): 549 (M + H)⁺.

### Example 2. (Compound No. 2) N-(4-methyl-3-(2-((6-methylpyridin-3-yl)amino)-8-vinylpyrido[3,4-d]pyrimidin-6-yl)phenyl)-3-(trifluoromethyl)benzamide

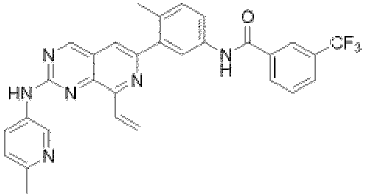

¹H-NMR: (400 MHz, CDCl₃): δ (ppm) : 9.18 (1H, s), 9.56 (1H, s), 8.21 (2H, m), 8.15 (1H, d, *J =* 8.4 Hz), 8.0 (1H, d, *J* = 8.5 Hz), 7.99 (2H, m), 7.88 (1H, d, *J* = 8.4 Hz), 7.60 (3H,m), 7.45 (1H, d, *J* = 8.5 Hz), 7.32 (1H, d, *J* = 8.5 Hz), 6.78 (1H, d, *J* = 8.7 Hz), 5.74 (1H, m), 2.59 (3H, s), 2.48 (3H, s). LCMS (ESI): 541 (M + H)⁺.

### Example 3. (Compound No. 3) N-(3-(8-ethyl-2-((6-methylpyridin-3-yl)amino)pyrido[3,4-d]pyrimidin-6-yl)-4-methylphenyl)-3-(trifluoromethyl)benzamide

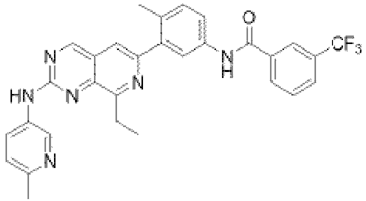

¹H-NMR: (400 MHz, CDCl₃): δ (ppm) : 9.18 (1H, s), 8.96 (1H, s), 8.29 (1H, d), 8.26 (1H, s), 8.15 (1H, d), 7.90 (1H, s), 7.83 (2H, m), 7.66 (3H, m), 7.44 (1H, s), 7.35 (1H, d), 7.33(1H, d), 3.46 (2H, q), 2.59 (3H, s), 2.44 (3H, s), 1.5 (3H, t). LCMS (ESI): 543 (M + H)⁺.

### Example 4. (Compound No. 4) N-(4-methyl-3-(2-((6-methylpyridin-3-yl)amino)pyrido[3,4-d]pyrimidin-6-yl)phenyl)-3-(trifluoromethyl)benzamide

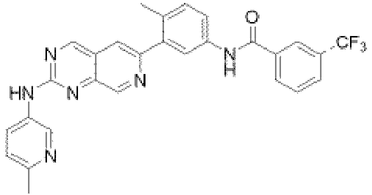

¹H-NMR: (400 MHz, DMSO-*d*₆) : δ (ppm) : 10.54 (1H, s), 10.30 (1H, s), 9.54 (1H, s), 9.21(1H, s), 8.99 (1H, brs), 8.31 (3H, m), 7.98 (3H, m), 7.79 (2H, d, *J* = 8.4 Hz), 7.28 (1H,d, *J* = 8.5 Hz), 7.25 (1H, d, *J* = 8.5 Hz), 2.45 (3H, s), 2.38 (3H, s). LCMS (ESI): 515 (M + H)⁺.

### Example 5. (Compound No. 5) N-(3-(2-((2-hydroxyethyl)amino)-8-methoxypyrido[3,4-d]pyrimidin-6-yl)-4-methylphenyl)-3-(trifluoromethyl)benzamide

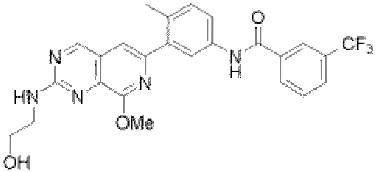

LCMS (ESI): 498 (M + H)⁻.

### Example 6. (Compound No. 6) N-(3-(2-(cyclopropylamino)-8-methoxypyrido[3,4-d]pyrimidin-6-yl)-4-methylphenyl)-3-(trifluoromethyl)benzamide

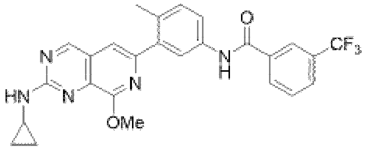

LCMS (ESI): 494 (M + H) ⁻.

### Example 7. (Compound No. 7) N-(3-(8-methoxy-2-morpholinopyrido[3,4-d]pyrimidin-6-yl)-4-methylphenyl)-3-(trifluoromethyl)benzamide

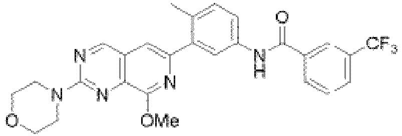

LCMS (ESI): 524 (M + H)⁻.

### Example 8. (Compound No. 8) N-(3-(8-methoxy-2-((6-methylpyridin-3-yl)amino)pyrido[3,4-d]pyrimidin-6-yl)-4-methylphenyl)-3-(trifluoromethyl)benzamide

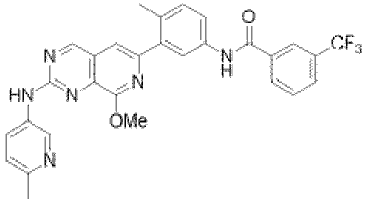

LCMS (ESI): 545 (M + H)⁻.

### Example 9. (Compound No. 9) N-(3-(8-methoxy-2-((6-morpholinopyridin-3-yl)amino)pyrido[3,4-d]pyrimidin-6-yl)-4-methylphenyl)-3-(trifluoromethyl)benzamide

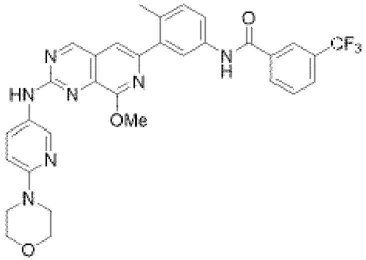

LCMS (ESI): 616 (M + H)⁻.

### Example 10. (Compound No. 10) N-(3-(8-methoxy-2-((2-methoxy-4-morpholinophenyl)amino)pyrido[3,4-d]pyrimidin-6-yl)-4-methylphenyl)-3-(trifluoromethyl)benzamide

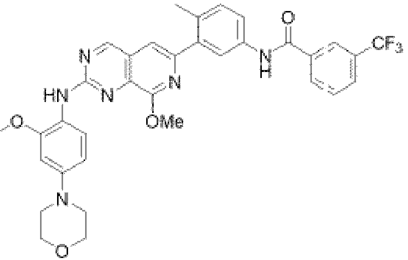

LCMS (ESI): 645 (M + H) +.

### Example 11. (Compound No. 11) N-(3-(2-((4-(4-ethylpiperazin-1-yl)phenyl)amino)-8-methoxypyrido[3,4-d]pyrimidin-6-yl)-4-methylphenyl)-3-(trifluoromethyl)benzamide

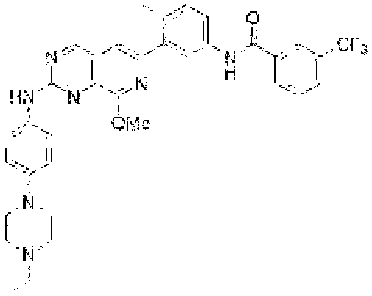

LCMS (ESI): 642 (M + H)⁻.

### Example 12. (Compound No. 12) N-(4-methyl-3-(2-((6-methylpyridin-3-yl)amino)pyrido[3,4-d]pyrimidin-6-yl)phenyl)-4-(morpholinemethyl)-3-(trifluoromethyl)benzamide

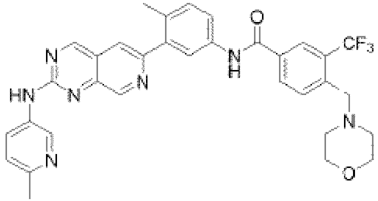

¹H-NMR: (400 MHz, DMSO-*d*₆) : δ (ppm): 9.82 (1H, s), 9.48 (1H, s), 9.29 (1H, s), 9.24 (1H, s), 9.07 (1H, s), 8.48 (1H, d), 8.34 (1H, s), 8.29 (1H, d), 8.05 (2H, m), 7.96 (1H, s), 7.85 (1H, d), 7.34 (1H, d), 7.28 (1H, d), 3.75 (2H, s), 3.67 (4H, m), 2.54 (4H, m), 2.51 (3H, s), 2.44 (3H, s). LCMS (ESI): 614 (M + H)⁺.

### Example 13. (Compound No. 13) 3-(4-methyl-1H-imidazol-1-yl)-N-(4-methyl-3-(2-((6-methylpyridin-3-yl)amino)pyrido[3,4-d]pyrimidin-6-yl)phenyl)-5-(trifluoromethyl)benzamide

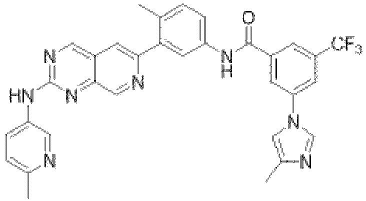

LCMS (ESI): 595 (M + H)⁻.

### Example 14. (Compound No. 14) N-(4-methyl-3-(2-((6-methylpyridin-3-yl)amino)pyrido[3,4-d]pyrimidin-6-yl)phenyl)-3-morpholino-5-(trifluoromethyl)benzamide

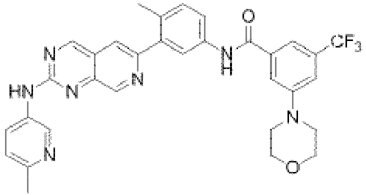

¹H-NMR: (400 MHz, DMSO-*d*₆) : δ (ppm) : 9.77 (1H, s), 9.46 (1H, s), 9.29 (1H, s), 9.23 (1H, s), 9.07 (1H, s), 8.47 (1H, d), 8.00 (1H, s), 7.94 (1H, s), 7.82 (2H, m), 7.73 (1H, s),7.40 (1H, s), 7.32 (1H, d), 7.27 (1H, d), 3.83 (4H, m), 3.45 (4H, m), 2.50 (3H, s), 2.43 (3H, s). LCMS (ESI): 600 (M + H)⁺.

### Example 15. (Compound No. 15) 4-(2,4-dimethyl-1H-imidazo-1-yl)-N-(4-methyl-3-(2-((6-methylpyridin-3-yl)amino)pyrido[3,4-d]pyrimidin-6-yl)phenyl)-3-(trifluoromethyl)benzamide

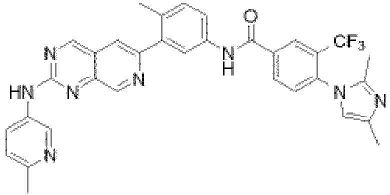

LCMS (ESI): 609 (M + H)⁻.

### Example 16. (Compound No. 16) 4-(4-hydroxypiperidin-1-yl)-N-(4-methyl-3-(2-((6-methylpyridin-3-yl)amino)pyrido[3,4-d]pyrimidin-6-yl)phenyl)-3-(trifluoromethyl)benzamide

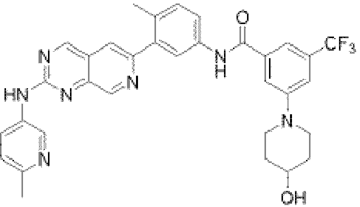

LCMS (ESI): 614 (M + H) ⁻.

### Example 17. (Compound No. 17) N-(3-(2-aminopyrido[3,4-d]pyrimidin-6-yl)-4-methylphenyl)-3-(trifluoromethyl)benzamide

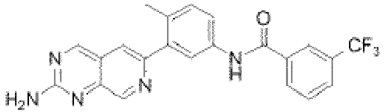

¹H-NMR: (400 MHz, DMSO-*d*₆) : δ (ppm) : 9.83 (1H, brs), 9.33 (1H, s), 9.01 (1H, s), 8.35 (1H, s), 8.34 (1H, d, *J* = 8.8 Hz), 8.00 (1H, s), 7.94 (1H, d, *J* = 7.6 Hz), 7.82 (3H, m),7.32 (1H, d, *J* = 8.4 Hz), 6.59 (2H, brs), 2.42 (3H, s). LCMS (ESI): 424 (M + H)⁺.

### Example 18. (Compound No. 18) N-(3-(2-((2-hydroxyethyl)amino)pyrido[3,4-d]pyrimidin-6-yl)-4-methylphenyl)-3-(trifluoromethyl)benzamide

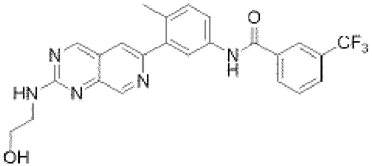

¹H-NMR: (400 MHz, DMSO-*d*₆) : δ (ppm) : 10.51 (1H, s), 9.32 (1H, brs), 9.00 (1H, s), 8.33 (1H, s), 8.26 (1H, d, *J* = 7.6 Hz), 7.94 (2H, m), 7.86 (1H, s), 7.77 (3H, m), 7.33 (1H, d, *J* = 8.4 Hz), 4.76 (1H, brs), 3.57 (2H, m), 3.50 (2H, m), 2.90 (1H, m), 2.37 (3H, s). LCMS (ESI): 468 (M + H)⁺.

### Example 19. (Compound No. 19) N-(3-(2-(cyclicpropylamino)-pyrido[3,4-d]pyrimidin-6-yl)-4-methylphenyl)-3-(trifluoromethyl)benzamide

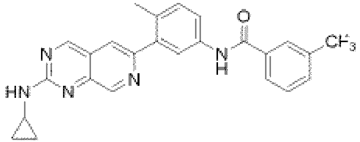

¹H-NMR: (400 MHz, DMSO-*d*₆) : δ (ppm) : 10.52 (1H, s), 9.32 (1H, brs), 9.06 (1H, s), 8.33 (1H, s), 8.28 (1H, d, *J* = 8.0 Hz), 8.04 (1H, s), 7.96 (2H, m), 7.94 (1H, s), 7.82 (2H, m),7.32 (1H, d, *J* = 8.4 Hz), 2.90 (1H, m), 2.37 (3H, s), 0.77 (2H, m), 0.58 (2H, m). LCMS (ESI): 464 (M + H)⁺.

### Example 20. (Compound No. 20) N-(4-methyl-3-(2-(phenylamino)pyrido[3,4-d]pyrimidin-6-yl)phenyl)-3-(trifluoromethyl)benzamide

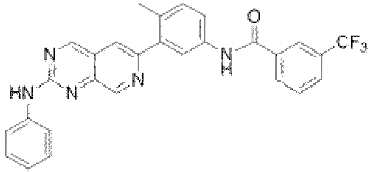

LCMS (ESI): 500 (M + H)⁻.

### Example 21. (Compound No. 21) N-(4-methyl-3-(2-((1-methyl-1H-pyrazo-4-yl)amino)pyrido[3,4-d]pyrimidin-6-yl)phenyl)-3-(trifluoromethyl)benzamide

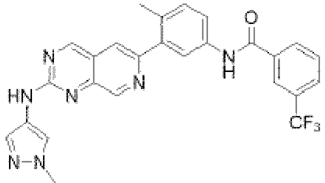

¹H-NMR: (400 MHz, acetone-*d*₆) : δ (ppm) : 9.83 (1H, s), 9.37 (1H, s), 9.22 (1H, s), 8.35 (3H, m), 8.02 (1H, s), 8.01 (1H, d), 7.93 (1H, s), 7.87 (2H, m), 7.76 (1H, s), 7.34 (1H,d), 3.93 (3H, s), 2.43 (3H, s). LCMS (ESI): 504 (M + H)⁺.

### Example 22. (Compound No. 22) N-(3-(2-((1,3-dimethyl-1H-pyrazol-5-yl)amino)pyrido[3,4-d]pyrimidin-6-yl)-4-methylphenyl)-3-(trifluoromethyl)benzamide

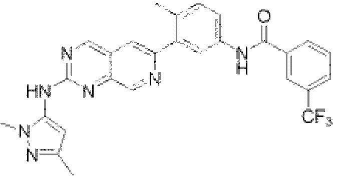

LCMS (ESI): 518 (M + H)⁻.

### Example 23. (Compound No. 23) N-(4-methyl-3-(2-((4-methylpyridin-3-yl)amino)pyrido[3,4-d]pyrimidin-6-yl)phenyl)-3-(trifluoromethyl)benzamide

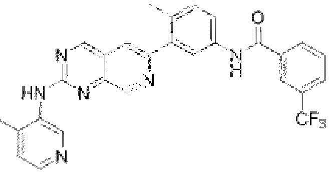

LCMS (ESI): 515 (M + H)⁻.

### Example 24. (Compound No. 24) N-(3-(2-((2,6-dimethylpyridin-3-yl)amino)pyrido[3,4-d]pyrimidin-6-yl)-4-methylphenyl)-3-(trifluoromethyl)benzamide

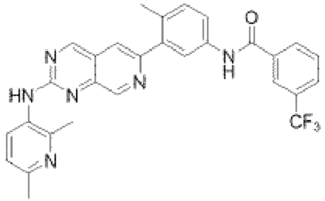

¹H-NMR: (400 MHz, acetone-*d*₆) : δ (ppm) : 9.84 (1H, s), 9.46 (1H, s), 9.08 (1H, s), 8.47 (1H, s), 8.36 (2H, m), 8.17 (1H, d), 8.03 (1H, s), 7.94 (2H, s), 7.82 (2H, m), 7.34 (1H,d), 7.16 (1H, d), 2.57 (3H, s), 2.50 (3H, s), 2.43 (3H, s). LCMS (ESI): 529 (M + H)⁺.

### Example 25. (Compound No. 25) N-(3-(2-((4-(4-ethylpiperazin-1-yl)phenyl)amino)pyrido[3,4-d]pyrimidin-6-yl)-4-methylphenyl)-3-(trifluoromethyl)benzamide

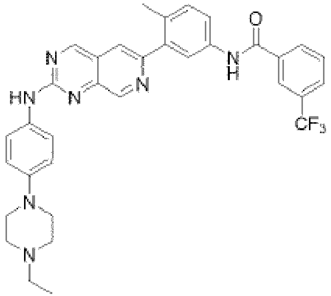

LCMS (ESI): 612 (M + H)⁻.

### Example 26. (Compound No. 26) N-(3-(2-((3-(4-ethylpiperazin-1-yl)phenyl)amino)pyrido[3,4-d]pyrimidin-6-yl)-4-methylphenyl)-3-(trifluoromethyl)benzamide

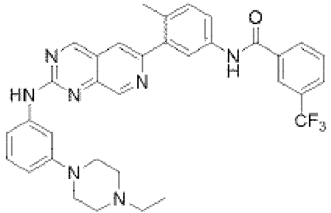

LCMS (ESI): 612 (M + H)⁻.

### [Experimental Examples]

### Experimental Example 1. Measurement of kinase inhibitory activity

In order to measure the protein kinase inhibitory activity (% inhibition) of the compound of the present invention, biochemical assay was performed in the full kinase panel.

As a test compound, Compound No. 4 was used. The percent inhibition of each kinase when treated with the test compound at a single concentration of 1 µM was measured, and the residual enzyme activity values (%) of the kinases were calculated. Kinases whose calculated residual enzyme activity values (%) was 30% or less (i.e., kinases inhibited by 70% or more) are as follows:

### <Kinases inhibited by 70% or more>

ABL1, ABL2/ARG, ACK1, ARAF, BLK, BMX/ETK, BRAF, BRK, BTK, c-Kit, c-Src, CSK, DDR1, DDR2, EPHA1, EPHA2, EPHA3, EPHA4, EPHA5, EPHA6, EPHA7, EPHA8, EPHB1, EPHB2, EPHB3, EPHB4, ERBB2/HER2, ERBB4/HER4, FGFR1, FGFR2, FGFR3, FGR, FLT1/VEGFR1, FLT4/VEGFR3, FMS, FRK/PTK5, FYN, GCK/MAP4K2, HCK, HGK/MAP4K4,JAK1, JAK2, KDR/VEGFR2, KHS/MAP4K5, LCK, LYN, LYN B, MEKK1, MLK4, MYLK3, NEK5, P38a/MAPK14, P38b/MAPK11, PDGFRa, PDGFRb, PEAK1, RET, RIPK2, RIPK4,SIK2, SRPK1, TAOK3/JIK, TLK1, TNK1, TXK, TYK2, YES/YES1, YSK4/MAP3K19, and ZAK/MLTK.

### Experimental Example 2. Inhibitory activities against GCK and ACK1 kinases

The inhibitory activities of the compounds of the present invention against two kinases, GCK and ACK1, were measured, and the IC₅₀ values thereof were calculated. The calculated IC₅₀ values are shown in Table 1 below. As a control, the following compound GNF7 was used.

### [Control: GNF 7]

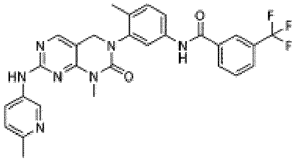

The calculated IC₅₀ values are shown in Table 1 below.

**[Table 1]**

| Test compound | Kinase inhibitory activity, IC₅₀ | |
|---|---|---|
| | ACK1 | GCK |
| GNF 7 | A | A |
| Compound No. 1 | B | D |
| Compound No. 3 | B | D |
| Compound No. 4 | A | B |
| Compound No. 18 | B | B |
| Compound No. 19 | B | B |
| [Classification of IC₅₀] A: less than 0.1 µM, B: 0.1 to 1.0 µM, C: 1.0 µM to 10.0 µM, D: more than 10 µM | | |

### Experimental Example 3. Inhibitory activities against proliferation of OCI-AML3 (N-Ras Q61L) and Ba/F3 (N-Ras G12D)

The inhibitory activities of the compounds of the present invention against the proliferation of the mt-NRAS (G12D) Ba/F3 and OCI-AML3 (mt-NRAS) cell lines were measured and the GI₅₀ values thereof were calculated. The calculated GI₅₀ values are shown in Table 2 below.

**[Table 2]**

| Test compound | Proliferation inhibitory activity (GI₅₀, µM) | |
|---|---|---|
| | OCI-AML3 (N-Ras Q61L) | Ba/F3 (N-Ras G12D) |
| Control (GNF 7) | A | B |
| Compound No. 1 | A | A |
| Compound No. 2 | B | A |
| Compound No. 3 | B | B |
| Compound No. 4 | A | A |
| Compound No. 8 | A | B |
| Compound No. 12 | A | A |
| Compound No. 13 | A | A |
| Compound No. 14 | A | A |
| Compound No. 17 | A | A |
| Compound No. 18 | A | A |
| Compound No. 19 | A | A |
| Compound No. 23 | A | A |
| Compound No. 24 | A | A |
| [Classification of IC₅₀] A: less than 0.5 µM, B: 0.5 to 3.0 µM, C: 3.0 µM to 5.0 µM, D: more than 5.0 µM | | |

Referring to the results in Table 2, it can be seen that the compound of the present invention has inhibitory activity against the proliferation of the human acute myeloid leukemia cell line (OCI-AML3) having the NRAS mutant gene, and the effect thereof is remarkable. Therefore, it can be seen that the compound of the present invention is particularly effective as a therapeutic agent for acute myeloid leukemia (AML).

### Experimental Example 4. Inhibitory activity against proliferation of Ba/F3 (parental)

The inhibitory activities of the compounds of the present invention against the proliferation of the Ba/F3 (parental) cell line were measured and the GI₅₀ values thereof were calculated. The calculated GI₅₀ values are shown in Table 3 below.

**[Table 3]**

| Test compound | Proliferation inhibitory activity (GI₅₀, µM) |
|---|---|
| | Ba/F3 (parental) |
| Control (GNF 7) | B |
| Compound No. 1 | A |
| Compound No. 2 | B |
| Compound No. 3 | A |
| Compound No. 4 | D |
| Compound No. 8 | B |
| Compound No. 12 | A |
| Compound No. 13 | A |
| Compound No. 14 | C |
| Compound No. 17 | A |
| Compound No. 18 | A |
| Compound No. 19 | C |
| Compound No. 23 | D |
| Compound No. 24 | A |
| [Classification of IC₅₀] A: more than 5.0 µM, B: 3.0 to 5.0 µM, C: 0.5 µM to 3.0 µM, D: less than 0.5 µM | |

It can be seen that the inhibitory activity of the compound of the present invention against Ba/F3 (parental) is lower than that of the control, suggesting that the compound of the present invention has low cytotoxicity.

### [Formulation Examples]

Meanwhile, the novel compound according to the present invention may be formulated in various forms depending on the intended use thereof. The following exemplifies several formulation methods containing, as an active ingredient, the compound according to the present invention, but the scope of the present invention is not limited thereto.

### Formulation Example 1: Tablet (direct compression)

5.0 mg of the active ingredient was sieved and then mixed with 14.1 mg of lactose, 0.8 mg of crospovidone USNF and 0.1 mg of magnesium stearate, and the mixture was compressed into a tablet.

### Formulation Example 2: Tablet (wet granulation)

5.0 mg of the active ingredient was sieved and then mixed with 16.0 mg of lactose and 4.0 mg of starch. 0.3 mg of polysorbate 80 was dissolved in pure water, and then a suitable amount of the solution was added to the mixture, followed by atomization to obtain fine particles. After drying, the fine particles were sieved and then mixed with 2.7 mg of colloidal silicon dioxide and 2.0 mg of magnesium stearate. The fine particles were compressed into a tablet.

### Formulation Example 3: Powder and capsule

5.0 mg of the active ingredient was sieved and then mixed with 14.8 mg of lactose, 10.0 mg of polyvinyl pyrrolidone and 0.2 mg of magnesium stearate. A No. 5 hard gelatin capsule was filled with the mixture using a suitable device.

### Formulation Example 4: Formulation for injection

A formulation for injection containing 100 mg of the active ingredient, 180 mg of mannitol, 26 mg of Na₂HPO₄•12H₂O and 2,974 mg of distilled water was prepared.

Although the embodiments of the present invention have been described above, those skilled in the art to which the present invention pertains will understand that the present invention may be embodied in other specific forms without departing from the scope of the invention defined in the appended claims. Therefore, it should be understood that the embodiments described above are illustrative in all respects, not restrictive.

## Claims

1. A compound selected from the group consisting of the following Compound Nos. 1 to 26:
(Compound No. 1): *N*-(3-(8-chloro-2-((6-methylpyridin-3-yl)amino)pyrido[3,4-*d*]pyrimidin-6-yl)-4-methylphenyl)-3-(trifluoromethyl)benzamide;
(Compound No. 2): *N*-(4-methyl-3-(2-((6-methylpyridin-3-yl)amino)-8-vinylpyrido[3,4-*d*]pyrimidin-6-yl)phenyl)-3-(trifluoromethyl)benzamide;
(Compound No. 3): *N*-(3-(8-ethyl-2-((6-methylpyridin-3-yl)amino)pyrido[3,4-*d*]pyrimidin-6-yl)-4-methylphenyl)-3-(trifluoromethyl)benzamide;
(Compound No. 4): *N*-(4-methyl-3-(2-((6-methylpyridin-3-yl)amino)pyrido[3,4-*d*]pyrimidin-6-yl)phenyl)-3-(trifluoromethyl)benzamide;
(Compound No. 5): *N*-(3-(2-((2-hydroxyethyl)amino)-8-methoxypyrido[3,4-*d*]pyrimidin-6-yl)-4-methylphenyl)-3-(trifluoromethyl)benzamide;
(Compound No. 6): *N*-(3-(2-(cyclopropylamino)-8-methoxypyrido[3,4-*d*]pyrimidin-6-yl)-4-methylphenyl)-3-(trifluoromethyl)benzamide;
(Compound No. 7): *N*-(3-(8-methoxy-2-morpholinopyrido[3,4-d]pyrimidin-6-yl)-4-methylphenyl)-3-(trifluoromethyl)benzamide;
(Compound No. 8): *N*-(3-(8-methoxy-2-((6-methylpyridin-3-yl)amino)pyrido[3,4-*d*]pyrimidin-6-yl)-4-methylphenyl)-3-(trifluoromethyl)benzamide;
(Compound No. 9): *N*-(3-(8-methoxy-2-((6-morpholinopyridin-3-yl)amino)pyrido[3,4-*d*]pyrimidin-6-yl)-4-methylphenyl)-3-(trifluoromethyl)benzamide;
(Compound No. 10): *N*-(3-(8-methoxy-2-((2-methoxy-4-morpholinophenyl)amino)pyrido[3,4-*d*]pyrimidin-6-yl)-4-methylphenyl)-3-(trifluoromethyl)benzamide;
(Compound No. 11): *N*-(3-(2-((4-(4-ethylpiperazin-1-yl)phenyl)amino)-8-methoxypyrido[3,4-*d*]pyrimidin-6-yl)-4-methylphenyl)-3-(trifluoromethyl)benzamide;
(Compound No. 12): *N*-(4-methyl-3-(2-((6-methylpyridin-3-yl)amino)pyrido[3,4-*d*]pyrimidin-6-yl)phenyl)-4-(morpholinemethyl)-3-(trifluoromethyl)benzamide;
(Compound No. 13): 3-(4-methyl-1*H*-imidazol-1-yl)-*N*-(4-methyl-3-(2-((6-methylpyridin-3-yl)amino)pyrido[3,4-*d*]pyrimidin-6-yl)phenyl)-5-(trifluoromethyl)benzamide;
(Compound No. 14): *N*-(4-methyl-3-(2-((6-methylpyridin-3-yl)amino)pyrido[3,4-*d*]pyrimidin-6-yl)phenyl)-3-morpholino-5-(trifluoromethyl)benzamide;
(Compound No. 15): 4-(2,4-dimethyl-1*H*-imidazo-1-yl)-*N*-(4-methyl-3-(2-((6-methylpyridin-3-yl)amino)pyrido[3,4-*d*]pyrimidin-6-yl)phenyl)-3-(trifluoromethyl)benzamide;
(Compound No. 16): 4-(4-hydroxypiperidin-1-yl)-*N-*(4-methyl-3-(2-((6-methylpyridin-3-yl)amino)pyrido[3,4-*d*]pyrimidin-6-yl)phenyl)-3-(trifluoromethyl)benzamide;
(Compound No. 17): *N*-(3-(2-aminopyrido[3,4-*d*]pyrimidin-6-yl)-4-methylphenyl)-3-(trifluoromethyl)benzamide;
(Compound No. 18): *N*-(3-(2-((2-hydroxyethyl)amino)pyrido[3,4-*d*]pyrimidin-6-yl)-4-methylphenyl)-3-(trifluoromethyl)benzamide;
(Compound No. 19): *N*-(3-(2-(cyclicpropylamino)-pyrido[3,4-*d*]pyrimidin-6-yl)-4-methylphenyl)-3-(trifluoromethyl)benzamide;
(Compound No. 20): *N*-(4-methyl-3-(2-(phenylamino)pyrido[3,4-*d*]pyrimidin-6-yl)phenyl)-3-(trifluoromethyl)benzamide;
(Compound No. 21): *N*-(4-methyl-3-(2-((1-methyl-1*H*-pyrazo-4-yl)amino)pyrido[3,4-*d*]pyrimidin-6-yl)phenyl)-3-(trifluoromethyl)benzamide;
(Compound No. 22): *N*-(3-(2-((1,3-dimethyl-1*H*-pyrazol-5-yl)amino)pyrido[3,4-*d*]pyrimidin-6-yl)-4-methylphenyl)-3-(trifluoromethyl)benzamide;
(Compound No. 23): *N*-(4-methyl-3-(2-((4-methylpyridin-3-yl)amino)pyrido[3,4-d]pyrimidin-6-yl)phenyl)-3-(trifluoromethyl)benzamide;
(Compound No. 24): *N*-(3-(2-((2,6-dimethylpyridin-3-yl)amino)pyrido[3,4-*d*]pyrimidin-6-yl)-4-methylphenyl)-3-(trifluoromethyl)benzamide;
(Compound No. 25): *N*-(3-(2-((4-(4-ethylpiperazin-1-yl)phenyl)amino)pyrido[3,4-*d*]pyrimidin-6-yl)-4-methylphenyl)-3-(trifluoromethyl)benzamide; and
(Compound No. 26): *N*-(3-(2-((3-(4-ethylpiperazin-1-yl)phenyl)amino)pyrido[3,4-*d*]pyrimidin-6-yl)-4-methylphenyl)-3-(trifluoromethyl)benzamide,
or a pharmaceutically acceptable salt, hydrate or stereoisomer thereof.

2. The compound of claim 1, wherein the pharmaceutically acceptable salt is a salt of an inorganic acid or organic acid selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, and toluenesulfonic acid.

3. A pharmaceutical composition for preventing, alleviating or treating cancer containing the compound of claim 1 or 2 as an active ingredient.

4. The pharmaceutical composition of claim 3 for use in preventing, alleviating or treating cancer caused by NRAS mutation.

5. The pharmaceutical composition of claim 3, for application to a patient with NRAS mutation.

6. The pharmaceutical composition for use of claim 4, wherein the cancer is at least one selected from the group consisting of melanoma, colorectal cancer, lung cancer, thyroid cancer, multiple myeloma, and blood cancer.

7. The pharmaceutical composition for use of claim 4, wherein the cancer is acute myeloid leukemia (AML).

8. The pharmaceutical composition for use of claim 4, wherein the composition is administered to a patient with NRAS G12D.

9. The pharmaceutical composition of claim 3, wherein the active ingredient inhibits at least one protein kinase selected from among ABL1, ABL2/ARG, ACK1, ARAF, BLK, BMX/ETK, BRAF, BRK, BTK, c-Kit, c-Src, CSK, DDR1, DDR2, EPHA1, EPHA2, EPHA3, EPHA4, EPHA5, EPHA6,EPHA7, EPHA8, EPHB1, EPHB2, EPHB3, EPHB4, ERBB2/HER2, ERBB4/HER4, FGFR1, FGFR2, FGFR3, FGR, FLT1/VEGFR1, FLT4/VEGFR3, FMS, FRK/PTK5, FYN,GCK/MAP4K2, HCK, HGK/MAP4K4, JAK1, JAK2, KDR/VEGFR2, KHS/MAP4K5, LCK, LYN, LYN B, MEKK1, MLK4, MYLK3, NEK5, P38a/MAPK14, P38b/MAPK11,PDGFRa, PDGFRb, PEAK 1, RET, RIPK2, RIPK4, SIK2, SRPK1, TAOK3/JIK, TLK1, TNK1. TXK, TYK2, YES/YES1, YSK4/MAP3K19, and ZAK/MLTK.

## Patentansprüche

1. Verbindung, ausgewählt aus der Gruppe bestehend aus den folgenden Verbindungen Nr. 1 bis 26:
(Verbindung Nr. 1): *N*-(3-(8-Chlor-2-((6-methylpyridin-3-yl)amino)pyrido[3,4-*d*]pyrimidin-6-yl)-4-methylphenyl)-3-(trifluomethyl)benzamid;
(Verbindung Nr. 2): *N*-(4-Methyl-3-(2-((6-methylpyridin-3-yl)amino)-8-vinylpyrido[3,4-*d*]pyrimidin-6-yl)phenyl)-3-(trifluormethyl)benzamid;
(Verbindung Nr. 3): *N*-(3-(8-Ethyl-2-((6-methylpyridin-3-yl)amino)pyrido[3,4-*d*]pyrimidin-6-yl)-4-methylphenyl)-3-(trifluormethyl)benzamid;
(Verbindung Nr. 4): *N*-(4-Methyl-3-(2-((6-methylpyridin-3-yl)amino)pyrido[3,4-*d*]pyrimidin-6-yl)phenyl)-3-(trifluormethyl)benzamid;
(Verbindung Nr. 5): *N*-(3-(2-((2-Hydroxyethyl)amino)-8-methoxypyrido[3,4-*d*]pyrimidin-6-yl)-4-methylphenyl)-3-(trifluormethyl)benzamid;
(Verbindung Nr. 6): *N*-(3-(2-(Cyclopropylamino)-8-methoxypyrido[3,4-*d*]pyrimidin-6-yl)-4-methylphenyl)-3-(trifluormethyl)benzamid;
(Verbindung Nr. 7): *N*-(3-(8-Methoxy-2-morpholinopyrido[3,4-d]pyrimidin-6-yl)-4-methylphenyl)-3-(trifluormethyl)benzamid;
(Verbindung Nr. 8): *N*-(3-(8-Methoxy-2-((6-methylpyridin-3-yl)amino)pyrido[3,4-*d*]pyrimidin-6-yl)-4-methylphenyl)-3-(trifluormethyl)benzamid;
(Verbindung Nr. 9): *N*-(3-(8-Methoxy-2-((6-morpholinopyridin-3-yl)amino)pyrido[3,4-*d*]pyrimidin-6-yl)-4-methylphenyl)-3-(trifluormethyl)benzamid;
(Verbindung Nr. 10): *N*-(3-(8-Methoxy-2-((2-methoxy-4-morpholinophenyl)amino)pyrido[3,4-*d*]pyrimidin-6-yl)-4-methylphenyl)-3-(trifluormethyl)benzamid;
(Verbindung Nr. 11): *N*-(3-(2-((4-(4-Ethylpiperazin-1-yl)phenyl)amino)-8-methoxypyrido[3,4-*d*]pyrimidin-6-yl)-4-methylphenyl)-3-(trifluormethyl)benzamid;
(Verbindung Nr. 12): *N*-(4-Methyl-3-(2-((6-methylpyridin-3-yl)amino)pyrido[3,4-*d*]pyrimidin-6-yl)phenyl)-4-(morpholinemethyl)-3-(trifluormethyl)benzamid;
(Verbindung Nr. 13): 3-(4-methyl-1*H*-imidazol-1-yl)-*N*-(4-methyl-3-(2-((6-methylpyridin-3-yl)amino)pyrido[3,4-*d*]pyrimidin-6-yl)phenyl)-5-(trifluormethyl)benzamid;
(Verbindung Nr. 14): *N*-(4-Methyl-3-(2-((6-methylpyridin-3-yl)amino)pyrido[3,4-*d*]pyrimidin-6-yl)phenyl)-3-morpholino-5-(trifluormethyl)benzamid;
(Verbindung Nr. 15): 4-(2,4-Dimethyl-1*H*-imidazo-1-yl)-*N*-(4-methyl-3-(2-((6-methylpyridin-3-yl)amino)pyrido[3,4-*d*]pyrimidin-6-yl)phenyl)-3-(trifluormethyl)benzamid;
(Verbindung Nr. 16): 4-(4-Hydroxypiperidin-1-yl)-*N*-(4-methyl-3-(2-((6-methylpyridin-3-yl)amino)pyrido[3,4-*d*]pyrimidin-6-yl)phenyl)-3-(trifluormethyl)benzamid;
(Verbindung Nr. 17): *N*-(3-(2-Aminopyrido[3,4-*d*]pyrimidin-6-yl)-4-methylphenyl)-3-(trifluormethyl)benzamid;
(Verbindung Nr. 18): *N*-(3-(2-((2-Hydroxyethyl)amino)pyrido[3,4-*d*]pyrimidin-6-yl)-4-methylphenyl)-3-(trifluormethyl)benzamid;
(Verbindung Nr. 19): *N*-(3-(2-(Cyclopropylamino)-pyrido[3,4-*d*]pyrimidin-6-yl)-4-methylphenyl)-3-(trifluormethyl)benzamid;
(Verbindung Nr. 20): *N*-(4-Methyl-3-(2-(phenylamino)pyrido[3,4-d]pyrimidin-6-yl)phenyl)-3-(trifluormethyl)benzamid;
(Verbindung Nr. 21): *N*-(4-Methyl-3-(2-((1-methyl-1*H*-pyrazo-4-yl)amino)pyrido[3,4-*d*]pyrimidin-6-yl)phenyl)-3-(trifluormethyl)benzamid;
(Verbindung Nr. 22): *N*-(3-(2-((1,3-Dimethyl-1*H*-pyrazol-5-yl)amino)pyrido[3,4-*d*]pyrimidin-6-yl)-4-methylphenyl)-3-(trifluormethyl)benzamid;
(Verbindung Nr. 23): *N*-(4-Methyl-3-(2-((4-methylpyridin-3-yl)amino)pyrido[3,4-*d*]pyrimidin-6-yl)phenyl)-3-(trifluormethyl)benzamid;
(Verbindung Nr. 24): *N*-(3-(2-((2,6-Dimethylpyridin-3-yl)amino)pyrido[3,4-*d*]pyrimidin-6-yl)-4-methylphenyl)-3-(trifluormethyl)benzamid;
(Verbindung Nr. 25): *N*-(3-(2-((4-(4-Ethylpiperazin-1-yl)phenyl)amino)pyrido[3,4-d]pyrimidin-6-yl)-4-methylphenyl)-3-(trifluormethyl)benzamid und
(Verbindung Nr. 26): *N*-(3-(2-((3-(4-Ethylpiperazin-1-yl)phenyl)amino)pyrido[3,4-*d*]pyrimidin-6-yl)-4-methylphenyl)-3-(trifluormethyl)benzamid,
oder ein pharmazeutisch annehmbares Salz, Hydrat oder Stereoisomer davon.

2. Verbindung nach Anspruch 1, wobei das pharmazeutisch annehmbare Salz ein Salz einer anorganischen Säure oder organischen Säure ist, ausgewählt aus der Gruppe bestehend aus Salzsäure, Bromwasserstoffsäure, Schwefelsäure, Phosphorsäure, Salpetersäure, Essigsäure, Glykolsäure, Milchsäure, Brenztraubensäure, Malonsäure, Bernsteinsäure, Glutarsäure, Fumarsäure, Apfelsäure, Mandelsäure, Weinsäure, Zitronensäure, Ascorbinsäure, Palmitinsäure, Maleinsäure, Hydroxymaleinsäure, Benzoesäure, Hydroxybenzoesäure, Phenylessigsäure, Zimtsäure, Salicylsäure, Methansulfonsäure, Benzolsulfonsäure und Toluolsulfonsäure.

3. Pharmazeutische Zusammensetzung zur Vorbeugung, Linderung oder Behandlung von Krebs, die die Verbindung nach Anspruch 1 oder 2 als Wirkstoffbestandteil enthält.

4. Pharmazeutische Zusammensetzung nach Anspruch 3 zur Verwendung bei der Vorbeugung, Linderung oder Behandlung von Krebs, der durch eine NRAS-Mutation verursacht wird.

5. Pharmazeutische Zusammensetzung nach Anspruch 3 zur Anwendung bei einem Patienten mit NRAS-Mutation.

6. Pharmazeutische Zusammensetzung zur Verwendung nach Anspruch 4, wobei der Krebs mindestens einer aus der Gruppe bestehend aus Melanom, Darmkrebs, Lungenkrebs, Schilddrüsenkrebs, multiplem Myelom und Blutkrebs ist.

7. Pharmazeutische Zusammensetzung zur Verwendung nach Anspruch 4, wobei der Krebs akute myeloische Leukämie (AML) ist.

8. Pharmazeutische Zusammensetzung zur Verwendung nach Anspruch 4, wobei die Zusammensetzung einem Patienten mit NRAS G12D verabreicht wird.

9. Pharmazeutische Zusammensetzung nach Anspruch 3, wobei der Wirkstoff mindestens eine Proteinkinase hemmt, ausgewählt aus ABL1, ABL2/ARG, ACK1, ARAF, BLK, BMX/ETK, BRAF, BRK, BTK, c-Kit, c-Src, CSK, DDR1, DDR2, EPHA1, EPHA2, EPHA3, EPHA4, EPHA5, EPHA6,EPHA7, EPHA8, EPHB1, EPHB2, EPHB3, EPHB4, ERBB2/HER2, ERBB4/HER4, FGFR1, FGFR2, FGFR3, FGR, FLT1/VEGFR1, FLT4/VEGFR3, FMS, FRK/PTK5, FYN,GCK/MAP4K2, HCK, HGK/MAP4K4, JAK1, JAK2, KDR/VEGFR2, KHS/MAP4K5, LCK, LYN, LYN B, MEKK1, MLK4, MYLK3, NEK5, P38a/MAPK14, P38b/MAPK11,PDGFRa, PDGFRb, PEAK1, RET, RIPK2, RIPK4, SIK2, SRPK1, TAOK3/JIK, TLK1, TNK1, TXK, TYK2, YES/YES1, YSK4/MAP3K19 und ZAK/MLTK.

## Revendications

1. Composé choisi dans le groupe constitué par les composés n° 1 à 26 suivants :
(Composé n° 1) : le *N*-(3-(8-chloro-2-((6-méthylpyridin-3-yl)amino)pyrido[3,4-*d*]pyrimidin-6-yl)-4-méthylphényl)-3-(trifluorométhyl)benzamide ;
(Composé n° 2) : le *N*-(4-méthyl-3-(2-((6-méthylpyridin-3-yl)amino)-8-vinylpyrido[3,4-*d*]pyrimidin-6-yl)phényl)-3-(trifluorométhyl)benzamide ;
(Composé n° 3) : le *N*-(3-(8-éthyl-2-((6-méthylpyridin-3-yl)amino)pyrido[3,4-*d*]pyrimidin-6-yl)-4-méthylphényl)-3-(trifluorométhyl)benzamide ;
(Composé n° 4) : le *N*-(4-méthyl-3-(2-((6-méthylpyridin-3-yl)amino)pyrido[3,4-*d*]pyrimidin-6-yl)phényl)-3-(trifluorométhyl)benzamide ;
(Composé n° 5) : le *N*-(3-(2-((2-hydroxyéthyl)amino)-8-méthoxypyrido[3,4-*d*]pyrimidin-6-yl)-4-méthylphényl)-3-(trifluorométhyl)benzamide ;
(Composé n° 6) : le *N*-(3-(2-(cyclopropylamino)-8-méthoxypyrido[3,4-*d*]pyrimidin-6-yl)-4-méthylphényl)-3-(trifluorométhyl)benzamide ;
(Composé n° 7) : le *N*-(3-(8-méthoxy-2-morpholinopyrido[3,4-*d*]pyrimidin-6-yl)-4-méthylphényl)-3-(trifluorométhyl)benzamide ;
(Composé n° 8) : le *N*-(3-(8-méthoxy-2-((6-méthylpyridin-3-yl)amino)pyrido[3,4-*d*]pyrimidin-6-yl)-4-méthylphényl)-3-(trifluorométhyl)benzamide ;
(Composé n° 9): le *N*-(3-(8-méthoxy-2-((6-morpholinopyridin-3-yl)amino)pyrido[3,4-*d*]pyrimidin-6-yl)-4-méthylphényl)-3-(trifluorométhyl)benzamide ;
(Composé n° 10) : le *N*-(3-(8-méthoxy-2-((2-méthoxy-4-morpholinophényl)amino)pyrido[3,4-*d*]pyrimidin-6-yl)-4-méthylphényl)-3-(trifluorométhyl)benzamide ;
(Composé n° 11) : le *N*-(3-(2-((4-(4-éthylpipérazin-1-yl)phényl)amino)-8-méthoxypyrido[3,4-*d*]pyrimidin-6-yl)-4-méthylphényl)-3-(trifluorométhyl)benzamide ;
(Composé n° 12) : le *N*-(4-méthyl-3-(2-((6-méthylpyridin-3-yl)amino)pyrido[3,4-*d*]pyrimidin-6-yl)phényl)-4-(morpholineméthyl)-3-(trifluorométhyl)benzamide ;
(Composé n° 13) : le 3-(4-méthyl-1*H-*imidazol-1-yl)-*N*-(4-méthyl-3-(2-((6-méthylpyridin-3-yl)amino)pyrido[3,4-*d*]pyrimidin-6-yl)phényl)-5-(trifluorométhyl)benzamide ;
(Composé n° 14) : le *N*-(4-méthyl-3-(2-((6-méthylpyridin-3-yl)amino)pyrido[3,4-*d*]pyrimidin-6-yl)phényl)-3-morpholino-5-(trifluorométhyl)benzamide ;
(Composé n° 15) : le 4-(2,4-diméthyl-1*H-*imidazo-1-yl)-*N-*(4-méthyl-3-(2-((6-méthylpyridin-3-yl)amino)pyrido[3,4-*d*]pyrimidin-6-yl)phényl)-3-(trifluorométhyl)benzamide ;
(Composé n° 16): le 4-(4-hydroxypipéridin-1-yl)-*N*-(4-méthyl-3-(2-((6-méthylpyridin-3-yl)amino)pyrido[3,4-*d*]pyrimidin-6-yl)phényl)-3-(trifluorométhyl)benzamide ;
(Composé n° 17) : le *N*-(3-(2-aminopyrido[3,4-*d*]pyrimidin-6-yl)-4-méthylphényl)-3-(trifluorométhyl)benzamide ;
(Composé n° 18) : le *N*-(3-(2-((2-hydroxyéthyl)amino)pyrido[3,4-*d*]pyrimidin-6-yl)-4-méthylphényl)-3-(trifluorométhyl)benzamide ;
(Composé n° 19) : le *N*-(3-(2-(cycliquepropylamino)-pyrido[3,4-*d*]pyrimidin-6-yl)-4-méthylphényl)-3-(trifluorométhyl)benzamide ;
(Composé n° 20) : le *N*-(4-méthyl-3-(2-(phénylamino)pyrido[3,4-*d*]pyrimidin-6-yl)phényl)-3-(trifluorométhyl)benzamide ;
(Composé n° 21) : le *N*-(4-méthyl-3-(2-((1-méthyl-1*H*-pyrazo-4-yl)amino)pyrido[3,4-*d*]pyrimidin-6-yl)phényl)-3-(trifluorométhyl)benzamide ;
(Composé n° 22) : le *N*-(3-(2-((1,3-diméthyl-1*H*pyrazol-5-yl)amino)pyrido[3,4-*d*]pyrimidin-6-yl)-4-méthylphényl)-3-(trifluorométhyl)benzamide ;
(Composé n° 23) : le *N*-(4-méthyl-3-(2-((4-méthylpyridin-3-yl)amino)pyrido[3,4-*d*]pyrimidin-6-yl)phényl)-3-(trifluorométhyl)benzamide ;
(Composé n° 24) : le *N*-(3-(2-((2,6-diméthylpyridin-3-yl)amino)pyrido[3,4-*d*]pyrimidin-6-yl)-4-méthylphényl)-3-(trifluorométhyl)benzamide ;
(Composé n° 25) : le *N*-(3-(2-((4-(4-éthylpipérazin-1-yl)phényl)amino)pyrido[3,4-*d*]pyrimidin-6-yl)-4-méthylphényl)-3-(trifluorométhyl)benzamide ; et
(Composé n° 26) : le *N*-(3-(2-((3-(4-éthylpipérazin-1-yl)phényl)amino)pyrido[3,4-*d*]pyrimidin-6-yl)-4-méthylphényl)-3-(trifluorométhyl)benzamide,
ou sel pharmaceutiquement acceptable de celui-ci, hydrate de celui-ci ou stéréoisomère de celui-ci.

2. Composé selon la revendication 1, dans lequel le sel pharmaceutiquement acceptable est un sel d'un acide inorganique ou d'un acide organique choisi dans le groupe constitué par l'acide chlorhydrique, l'acide bromhydrique, l'acide sulfurique, l'acide phosphorique, l'acide nitrique, l'acide acétique, l'acide glycolique, l'acide lactique, l'acide pyruvique, l'acide malonique, l'acide succinique, l'acide glutarique, l'acide fumarique, l'acide malique, l'acide mandélique, l'acide tartrique, l'acide citrique, l'acide ascorbique, l'acide palmitique, l'acide maléique, l'acide hydroxymaléique, l'acide benzoïque, l'acide hydroxybenzoïque, l'acide phénylacétique, l'acide cinnamique, l'acide salicylique, l'acide méthanesulfonique, l'acide benzènesulfonique et l'acide toluènesulfonique.

3. Composition pharmaceutique pour prévenir, soulager ou traiter le cancer, contenant le composé selon la revendication 1 ou 2 en tant que principe actif.

4. Composition pharmaceutique selon la revendication 3 pour une utilisation dans la prévention, le soulagement ou le traitement du cancer causé par la mutation de NRAS.

5. Composition pharmaceutique selon la revendication 3, pour une application à un patient présentant une mutation de NRAS.

6. Composition pharmaceutique pour une utilisation selon la revendication 4, dans laquelle le cancer est au moins un cancer choisi dans le groupe constitué par le mélanome, le cancer colorectal, le cancer du poumon, le cancer de la thyroïde, le myélome multiple et le cancer du sang.

7. Composition pharmaceutique pour une utilisation selon la revendication 4, dans laquelle le cancer est la leucémie myéloïde aiguë (LMA).

8. Composition pharmaceutique pour une utilisation selon la revendication 4, dans laquelle la composition est administrée à un patient présentant le NRAS G12D.

9. Composition pharmaceutique selon la revendication 3, dans laquelle le principe actif inhibe au moins une protéine kinase choisie parmi ABL1, ABL2/ARG, ACK1, ARAF, BLK, BMX/ETK, BRAF, BRK, BTK, c-Kit, c-Src, CSK, DDR1, DDR2, EPHA1, EPHA2, EPHA3, EPHA4, EPHA5, EPHA6,EPHA7, EPHA8, EPHB1, EPHB2, EPHB3, EPHB4, ERBB2/HER2, ERBB4/HER4, FGFR1, FGFR2, FGFR3, FGR, FLT1/VEGFR1, FLT4/VEGFR3, FMS, FRK/PTK5, FYN,GCK/MAP4K2, HCK, HGK/MAP4K4, JAK1, JAK2, KDR/VEGFR2, KHS/MAP4K5, LCK, LYN, LYN B, MEKK1, MLK4, MYLK3, NEK5, P38a/MAPK14, P38b/MAPK11, PDGFRa, PDGFRb, PEAK1, RET, RIPK2, RIPK4, SIK2, SRPK1, TAOK3/JIK, TLK1, TNK1, TXK, TYK2, YES/YES1, YSK4/MAP3K19, et ZAK/MLTK.
